# EUROPEAN PATENT APPLICATION

(11) **EP 4 643 884 A1**
(43) Date of publication of application: **05.11.2025**
(21) Application number: 23910373.2
(22) Date of filing: 22.12.2023
(51) Int. Cl.: A61K 47/68, C07D 491/22, A61P 35/00

(54) **ANTIBODY-DRUG CONJUGATE FROM N-HALOALKYL SUBSTITUTED CAMPTOTHECIN DERIVATIVE**

(30) Priority: 29.12.2022 CN 202211740110
(71) Applicant: Hangzhou Adcoris Biopharma Co., Ltd, Hangzhou, Zhejiang 310056 (CN)
(72) Inventor: HUANG, Yunsheng, Hangzhou, Zhejiang 310056 (CN); LI, Yaowu, Hangzhou, Zhejiang 310056 (CN); WANG, Feng, Hangzhou, Zhejiang 310056 (CN)
(74) Representative: Elzaburu S.L.P.
(86) International application number: PCT/CN2023/140874
(87) International publication number: WO 2024/140447

(57) **Abstract**

An oxycycloalkyl-substituted camptothecin derivative used as an antibody-drug conjugate represented by formula (I) in the drug toxicity portion. The antibody-drug conjugate has a significant antitumor effect.

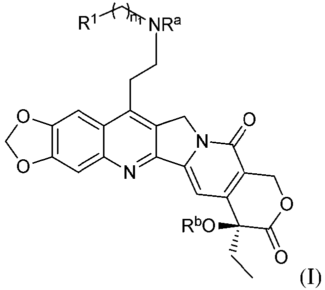

## Description

### TECHNICAL FIELD

The present disclosure relates to the medicinal field. Specifically, the present disclosure provides a series of antibody-drug conjugates (ADCs) of N-fluoroalkyl substituted camptothecin derivatives, preparation method thereof, and use thereof in the anti-tumor field.

### BACKGROUND

Camptothecin and derivatives thereof are a class of broad-spectrum antitumor drugs, and various camptothecin derivatives have been approved for the treatment of various cancers in many countries.

Antibody-drug conjugate (ADC) is formed by linking a bioactive small-molecule drug to a monoclonal antibody through a chemical linker, where the monoclonal antibody acts as a carrier to target and transport the small-molecule drug to target cells. In recent years, a variety of camptothecin derivatives have been used as small-molecule drugs in antibody-drug conjugates (ADCs), and have achieved very good anti-tumor effects. For example, Patent Document 201380053256.2 discloses a class of antibody-drug conjugates using irinotecan as the small-molecule drug payload, which shows obvious inhibitory effects on melanoma, non-small cell lung cancer, etc. However, the number of marketed ADC drugs is limited. At present, it is still necessary to explore the structure of camptothecin derivatives as ADC payloads and the structure of suitable linkers, and develop more antibody-drug conjugates with anti-tumor activity.

### SUMMARY

The purpose of the present disclosure is to provide an antibody-drug conjugate (ADC) using an haloalkyl substituted camptothecin derivative as the drug toxic moiety, particularly an ADC using a fluoroalkyl substituted camptothecin derivative as the drug toxic moiety. These antibody-drug conjugates exhibit significant anti-tumor effects.

One aspect of the present disclosure provides an antibody-drug conjugate represented by Formula (I), 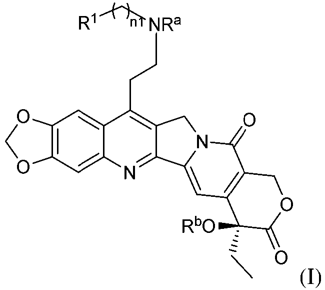 in the Formula,
R¹ is selected from halogen-substituted methyl;
R^{a} is selected from hydrogen and -L¹-NH-L^{p}-Z-Ab, R^{b} is selected from hydrogen, -L²-NH-L^{p}-Z-Ab and -L³-NH-Z-Ab, provided that R^{a}, R^{b} are not both hydrogen;
wherein,
L¹ is selected from -C(=O)-L¹¹-L¹²-L¹³- and -CH₂-NR²-L¹⁴-CHR³-;
L¹¹, L¹², L¹³ are each independently selected from-O-, C₁-C₃ alkylene and phenyl;
R² is selected from hydrogen, deuterium, C₁-C₆ alkyl, C₁-C₆ alkoxyl, acyl, sulfonyl;
L¹⁴ is selected from -C(=O)- and C₁-C₃ alkylene;
R³ is selected from hydrogen, deuterium, C₁-C₆ alkyl, C₁-C₆ alkoxyl and phenyl-substituted C₁-C₆ alkyl;
L² is selected from -C(=O)-NH-L²¹-CHR⁴-;
L²¹ is selected from -C(=O)- and -NH-C(=O)-;
R⁴ is selected from hydrogen, deuterium, C₁-C₆ alkyl and phenyl-substituted C₁-C₆ alkyl;
L³ is selected from -C(=O)-NR⁵-NR⁶-L³¹-L³²-;
R⁵, R⁶ are each independently selected from hydrogen, deuterium and C₁-C₆ alkyl;
L³¹ is selected from -C(=O), -(C₁-C₃ alkylene)-C(=O), -5-8 membered aryl-C(=O)-, -5-8 membered azaheteroaryl-C(=O)- and -O-(C₁-C₃ alkylene)-C(=O)-;
L³² is selected from -NH-(C₁-C₃ alkylene), -N(CH₃)₂-(C₁-C₃ alkylene) and -NH-(C₁-C₃ alkylene)-NH-;
L^{P} is a peptide residue consisting of 2 to 7 amino acids;
Z is selected from -L²-L^{j}-, wherein, L^{z} is selected from -C(=O)-C₁-C₈ alkylene, -C(=O)-CH₂O-(CH₂CH₂O)₂₋₅-CH₂CH₂NH- or -C(=O)-(CH₂CH₂O)₂₋₆-CH₂CH₂NH-, L^{j} is a linker that can conjugate to an antibody;
n₁ is selected from an integer of 1-3;
Ab is antibody.

In the antibody-drug conjugate represented by Formula (I), the drug antibody ratio (DAR) thereof is selected from 2-8, further can be 6-8 or 6.2-7.6, for example can be 6.2, 6.3, 6.5, 6.6, 6.8, 7.0, 7.1, 7.2, 7.3, 7.5 or 7.6.

One aspect of the present disclosure further provides an antibody-drug conjugate represented by Formula (IA), (IB) or (IC) below, 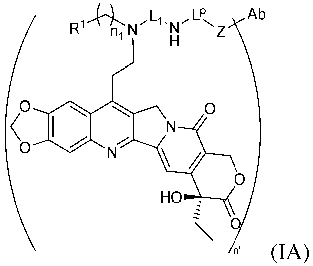 each group in the Formula is the same as defined above; 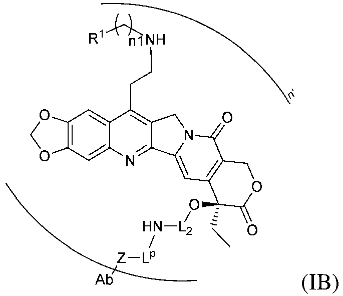 each group in the Formula is the same as defined above; 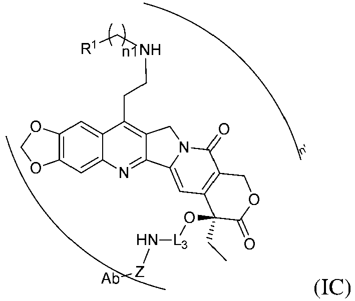
n' is selected from 2-8, further can be 6-8 or 6.2-7.6, for example can be 6.2, 6.3, 6.5, 6.6, 6.8, 7.0, 7.1, 7.2, 7.3, 7.5 or 7.6;
each group in the Formula is the same as defined above.

In some specific embodiments, R¹ is selected from methyl substituted by fluorine, chlorine or bromine.

In some specific embodiments, R¹ is selected from fluorine-substituted methyl.

In some specific embodiments, R¹ is selected from fluoromethyl, difluoromethyl and trifluoromethyl.

In some specific embodiments, R¹ is selected from difluoromethyl and trifluoromethyl.

In some specific embodiments, n₁ is 1.

In some specific embodiments, L¹¹, L¹², L¹³ are each independently selected from-O-, methylene and phenyl.

In some specific embodiments, L¹¹-L¹²-L¹³ is selected from -O-CH₂-phenyl- and -CH₂-O-CH₂-.

In some specific embodiments, R² is selected from hydrogen, deuterium, C₁-C₆ alkyl, C₁-C₆ alkoxyl, -C(=O)C₁-C₆ alkyl and -S(=O)₂C₁-C₆ alkyl.

In some specific embodiments, R² is selected from hydrogen, deuterium, C₁-C₃ alkyl, C₁-C₃ alkoxyl, -C(=O)C₁-C₃ alkyl and -S(=O)₂C₁-C₃ alkyl.

In some specific embodiments, R² is selected from hydrogen, deuterium, methyl, ethyl, methoxy, ethoxy, formyl, acetyl, methanesulfonyl and ethanesulfonyl.

In some specific embodiments, R² is selected from hydrogen, methyl, methoxy, formyl and methanesulfonyl.

In some specific embodiments, R² is hydrogen.

In some specific embodiments, L¹⁴ is selected from -C(=O)- and C₁-C₃ alkylene.

In some specific embodiments, L¹⁴ is selected from -C(=O)- and methylene.

In some specific embodiments, R³ is selected from hydrogen, deuterium, C₁-C₆ alkyl, C₁-C₆ alkoxyl and phenyl-substituted C₁-C₆ alkyl.

In some specific embodiments, R³ is selected from hydrogen, C₁-C₆ alkyl and phenyl-substituted C₁-C₆ alkyl.

In some specific embodiments, R³ is selected from hydrogen, C₁-C₄ alkyl and phenyl-substituted C₁-C₃ alkyl.

In some specific embodiments, R³ is selected from hydrogen, methyl, ethyl, propyl, isopropyl, butyl, isobutyl, phenylmethyl and phenylethyl.

In some specific embodiments, R³ is selected from hydrogen, methyl, isopropyl, isobutyl and phenylmethyl.

In some specific embodiments, R³ is hydrogen.

In some specific embodiments, L¹ is selected from -C(=O)-O-CH₂-phenyl-, -C(=O)-CH₂-O-CH₂-, -CH₂-NH-C(=O)-CH₂- and -CH₂-NH-CH₂-CH₂-.

In some specific embodiments, L¹ is selected from -C(=O)-O-CH₂-phenyl-, -C(=O)-CH₂-O-CH₂- and -CH₂-NH-C(=O)-CH₂-.

In some specific embodiments, L¹ is selected from -C(=O)-O-CH₂-phenyl- and -C(=O)-CH₂-O-CH₂-.

In some specific embodiments, R⁴ is selected from hydrogen, deuterium, C₁-C₆ alkyl and phenyl-substituted C₁-C₆ alkyl.

In some specific embodiments, R⁴ is selected from hydrogen, deuterium, C₁-C₃ alkyl and phenyl-substituted C₁-C₃ alkyl.

In some specific embodiments, R⁴ is selected from hydrogen, deuterium, methyl, isopropyl, isobutyl and phenylmethyl.

In some specific embodiments, R⁴ is selected from hydrogen.

In some specific embodiments, L² is selected from -C(=O)-NH-C(=O)-CH₂- and -CH₂-NH-NH-C(=O)-CH₂-.

In some specific embodiments, L² is -CH₂-NH-NH-C(=O)-CH₂-.

In some specific embodiments, R⁵, R⁶ are each independently selected from hydrogen, deuterium and C₁-C₃ alkyl.

In some specific embodiments, R⁵, R⁶ are each independently selected from hydrogen, deuterium and methyl.

In some specific embodiments, R⁵ is hydrogen, R⁶ is hydrogen.

In some specific embodiments, L³¹ is selected from -C(=O), -methylene-C(=O), -phenyl-C(=O)-, - pyridyl-C(=O)- and -O-CH₂-C(=O)-.

In some specific embodiments, L³² is selected from -NH-CH₂-CH₂-, -N(CH₃)₂-CH₂-CH₂- and -NH-CH₂-NH-.

In some specific embodiments, L³ is -C(=O)-NH-NH-phenyl-C(=O)-NH-CH₂-CH₂-.

In some specific embodiments, the amino acid is selected from phenylalanine (Phe), glycine (Gly), valine (Val), alanine (Ala), leucine (Leu), lysine (Lys), citrulline (Cit), and asparagine (Asn).

In some specific embodiments, L^{p} is selected from peptide residues consisting of 2-5 amino acids selected from phenylalanine (Phe), glycine (Gly), valine (Val), alanine (Ala), leucine (Leu), lysine (Lys), citrulline (Cit), and asparagine (Asn).

In some specific embodiments, L^{p} is selected from peptide residues consisting of 2-5 amino acids selected from phenylalanine (Phe), glycine (Gly), valine (Val), alanine (Ala), leucine (Leu) and lysine (Lys).

In some specific embodiments, L^{p} is selected from peptide residues consisting of 2, 3 or 4 amino acids selected from phenylalanine (Phe), glycine (Gly), valine (Val), alanine (Ala), leucine (Leu) and lysine (Lys).

In some specific embodiments, L^{p} is selected from -Val-Cit-, -Val-Ala-, -Gly-Val-Ala-, -Gly-Val-Ala-Gly-, -Gly-Lys-, -Gly-Gly-Lys-, -Gly-Gly-Lys-Gly-, -Val-Ala-, -Ala-Ala-Asn-, -Gly-Leu-, -Gly-Gly-Leu-, -Gly-Gly-Leu-Gly-, -Gly-Phe-, -Gly-Gly-Phe- and -Gly-Gly-Phe-Gly-.

In some specific embodiments, L^{p} is selected from 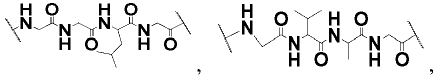 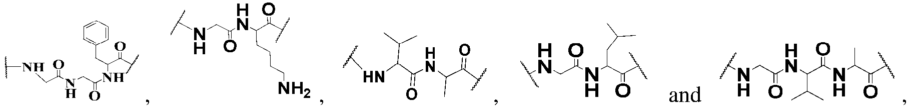 the carbonyl terminus thereof is linked to -NH- and the other terminus is linked to Z.

In some specific embodiments, L^{p} is selected from 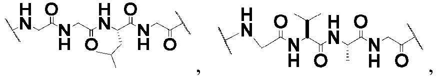 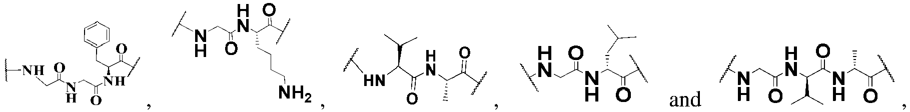 the carbonyl terminus thereof is linked to -NH- and the other terminus is linked to Z.

In some specific embodiments, L^{j} is selected from 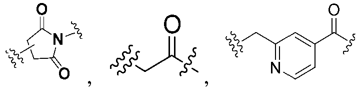 and 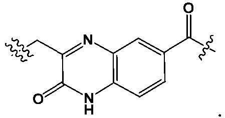 The position represented by " 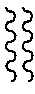 " is linked to the antibody, and the position represented by " 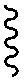 " is linked to the L^{Z} group.

In some specific embodiments, L^{z} is selected from -C(=O)-C₁-C₈ alkylene, -C(=O)-CH₂O-(CH₂CH₂O)₂₋₅-CH₂CH₂NH- and -C(=O)-(CH₂CH₂O)₂₋₆-CH₂CH₂NH-.

In some specific embodiments, L^{z} is selected from -C(=O)-C₁-C₆ alkylene, -C(=O)-CH₂O-(CH₂CH₂O)₂₋₃-CH₂CH₂NH- and -C(=O)-(CH₂CH₂O)₂₋₄-CH₂CH₂NH-.

In some specific embodiments, L^{z} is selected from -C(=O)-(CH₂CH₂O)₂-CH₂CH₂NH-, -C(=O)-CH₂O-CH₂CH₂O-CH₂CH₂NH- and -C(=O)-CH₂O-(CH₂CH₂O)₃-CH₂CH₂NH.

In some specific embodiments, Z is selected from 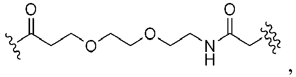 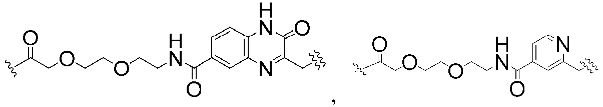 and 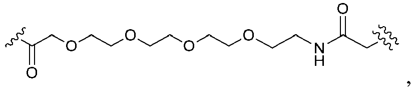 the position represented by " 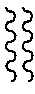 " is linked to the antibody, and the position represented by " 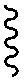 " is linked to the L^{p} group or L³ group.

In some specific embodiments, the antibody is an antibody against tumor-associated antigen.

In some specific embodiments, the antibody against tumor-associated antigen is selected from anti-Her2 antibody, anti-Trop2 antibody, anti-B7H3 antibody, anti-5T4 antibody, anti-Nectin-4 antibody, anti-CD20 antibody, and anti-ROR1 antibody.

In some specific embodiments, the antibody-drug conjugate represented by Formula (I) has a structure represented by Formula (I-1), Formula (I-1'), Formula (I-2') or Formula (I-2), 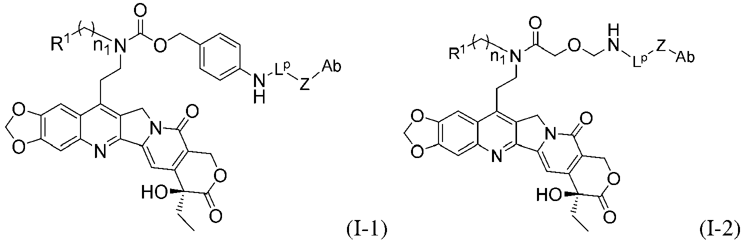 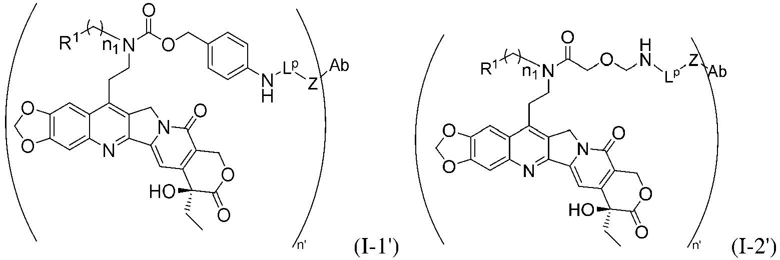 in the Formula, R¹, n₁, L^{p}, Z, Ab are each defined the same as the compound of Formula (I), n' is defined the same as above.

In some specific embodiments, the antibody-drug conjugate represented by Formula (I) has a structure represented by Formula (I-3), Formula (I-3'), Formula (I-4') or Formula (I-4), 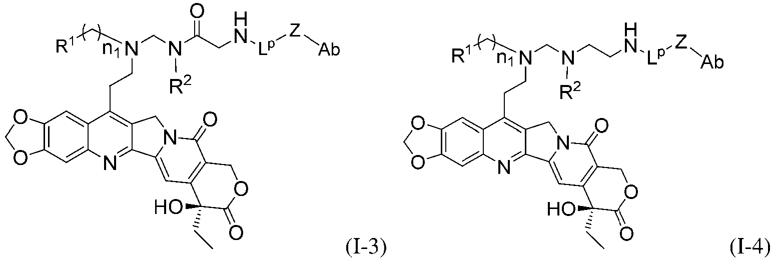 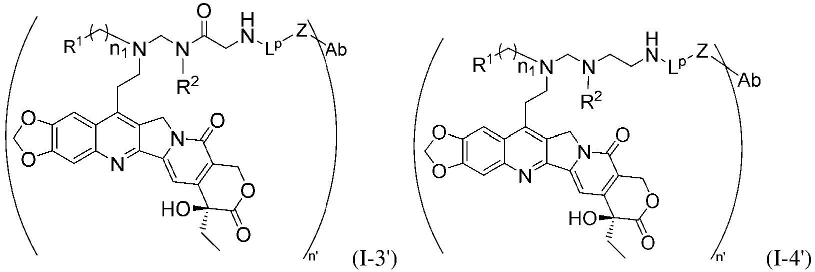 in the Formula, R¹, R², n₁, L^{p}, Z, Ab are each defined the same as the compound of Formula (I), n' is defined the same as above.

In some specific embodiments, the antibody-drug conjugate represented by Formula (I) has a structure represented by Formula (I-5) or Formula (I-5'), 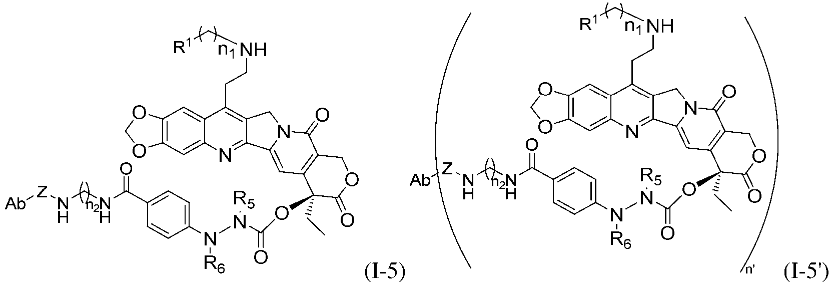 in the Formula, R¹, R⁵, R⁶, n₁, Ab are each defined the same as the compound of Formula (I), n' is defined the same as above.
n₂ is selected from 1 or 2.
Z is selected from -L^{z}-L^{j}-, wherein, L^{j} is selected from 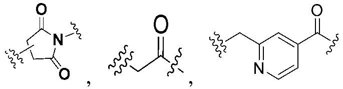 and 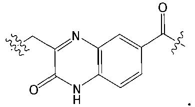 The position represented by " 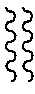 " is linked to the antibody, and the position represented by " 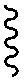 " is linked to the L^{Z} group;.

In some specific embodiments, L^{z} is selected from -C(=O)-CH₂O-(CH₂CH₂O)₁₋₅-CH₂CH₂NH-.

In some specific embodiments, L^{z} is selected from -C(=O)-CH₂O-(CH₂CH₂O)₃₋₅-CH₂CH₂NH-.

In some specific embodiments, L^{z} is selected from -C(=O)-CH₂O-(CH₂CH₂O)₃-CH₂CH₂NH-.

In some specific embodiments, Z is selected from 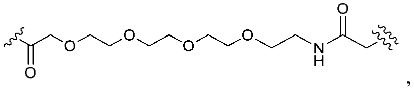 the position represented by " 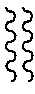 " is linked to the antibody, and the position represented by " 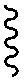 " is linked to the L^{p} group or L³ group.

In some specific embodiments, the antibody-drug conjugate represented by Formula (I) has a structure represented by Formula (I-6) or Formula (I-6'), 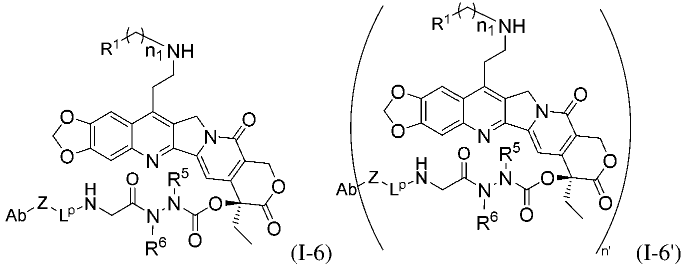 in the Formula, R¹, R⁵, R⁶, n₁, L^{p}, Z, Ab are each defined the same as the compound of Formula (I), n' is defined the same as above.

The present disclosure provides the following antibody-drug conjugate: 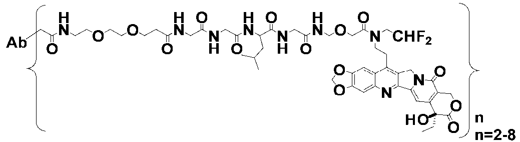 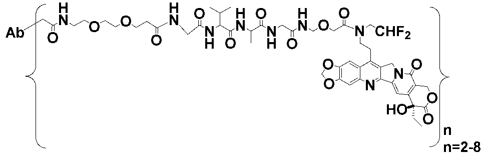 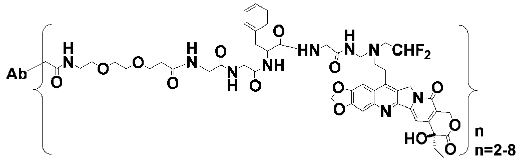 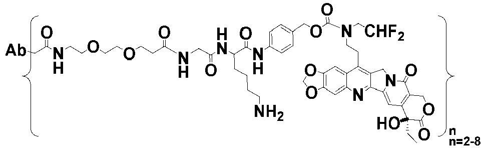 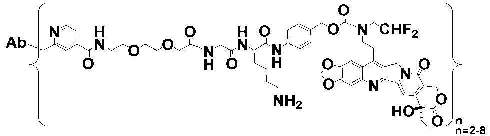 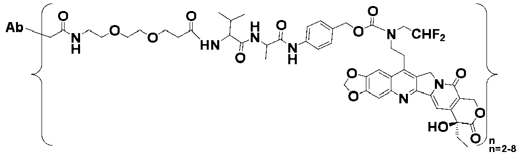 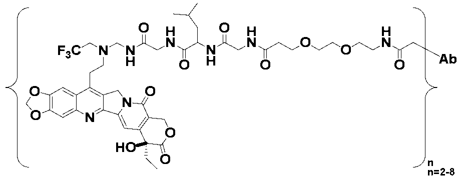 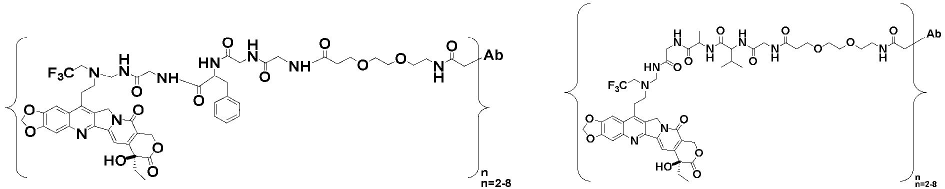 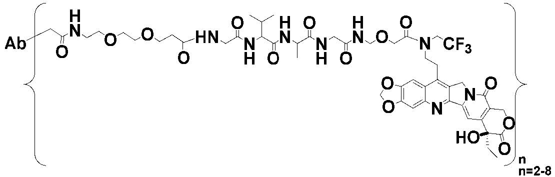 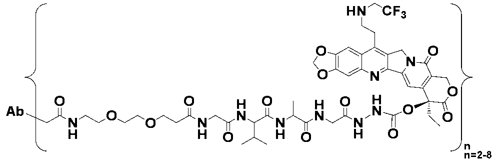 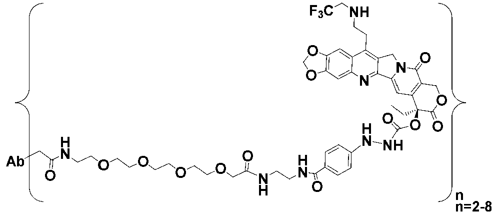 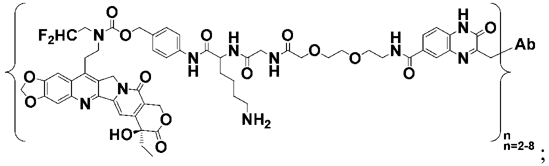 wherein, Ab is defined the same as the compound of Formula (I).

In some specific embodiments, Ab is selected from antibodies against tumor-associated antigens, and further selected from anti-Her2 antibodies, anti-Trop2 antibodies, anti-B7H3 antibodies, anti-5T4 antibodies, anti-Nectin-4 antibodies, anti-CD20 antibodies and anti-ROR1 antibodies.

Preferably, Ab is the HS627 antibodies or the IP140B antibodies, wherein the heavy chain amino acid sequence of HS627 is as set forth in SEQ ID NO: 1, and the light chain amino acid sequence thereof is as set forth in SEQ ID NO: 2; the heavy chain amino acid sequence of the IP140B antibodies is as set forth in SEQ ID NO: 3, and the light chain amino acid sequence thereof is as set forth in SEQ ID NO: 4.

In some specific embodiments, n is selected from 6-8, further can be 6.2-7.6, for example can be 6.2, 6.3, 6.5, 6.6, 6.8, 7.0, 7.1, 7.2, 7.3, 7.5 or 7.6.

The present disclosure provides the following antibody-drug conjugate: 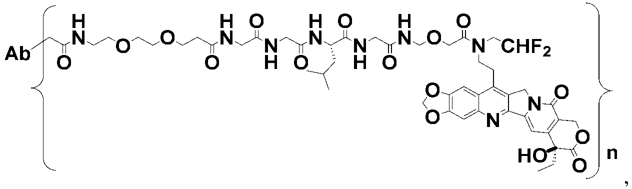 n is selected from 6.8-7.0. 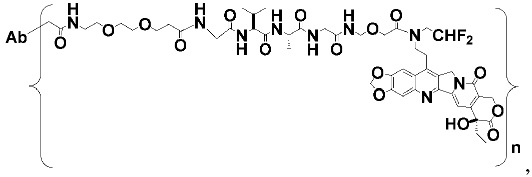 n is selected from 7.4-7.5. 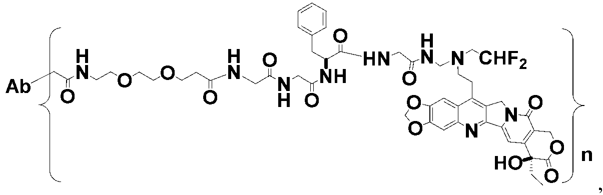 n is selected from 7.5-7.6. 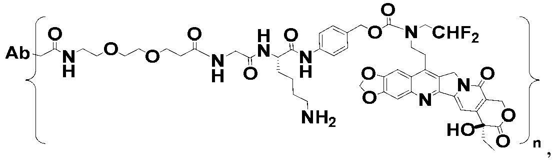 n is selected from 7.1-7.2. 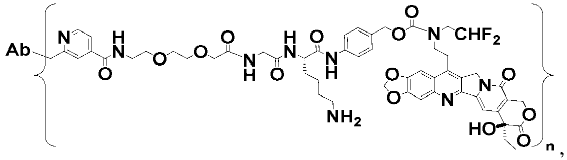 n is selected from 7.2-7.3. 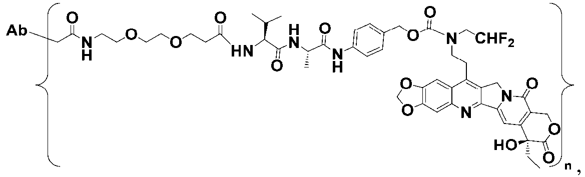 n is selected from 7.5-7.6. 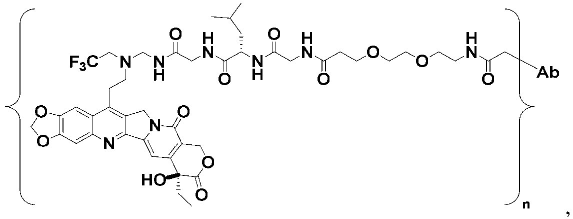 n is selected from 7.0-7.1. 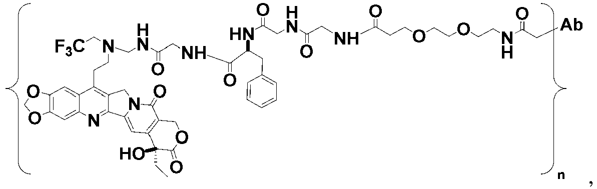 n is selected from 6.2-6.5. 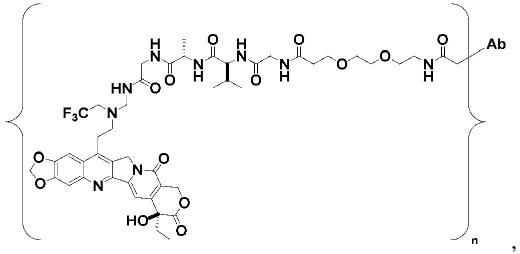 n is selected from 6.3-6.6. 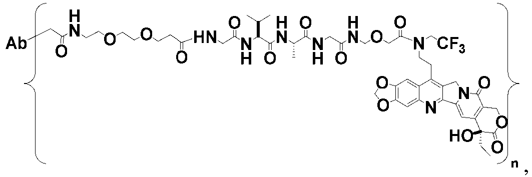 n is selected from 7.3-7.5. 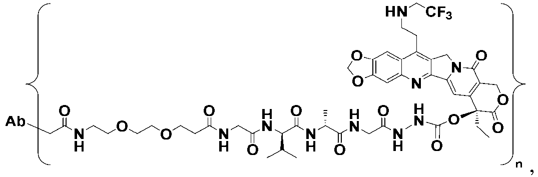 n is selected from 7.0-7.1. 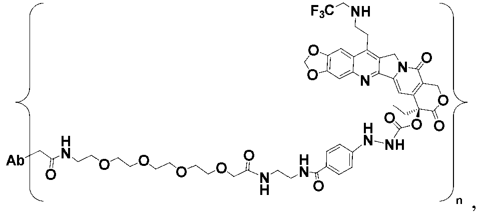 n is selected from 7.3-7.5. 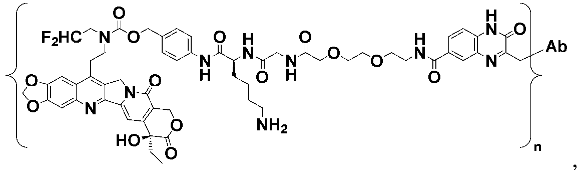 n is selected from 6.3-6.6.

Wherein, Ab is defined the same as the compound of Formula (I);
preferably, Ab is HS627 antibody or IP140B antibody, Ab is the HS627 antibodies or the IP140B antibodies, wherein the heavy chain amino acid sequence of HS627 is as set forth in SEQ ID NO: 1, and the light chain amino acid sequence thereof is as set forth in SEQ ID NO: 2; the heavy chain amino acid sequence of the IP140B antibodies is as set forth in SEQ ID NO: 3, and the light chain amino acid sequence thereof is as set forth in SEQ ID NO: 4. Another aspect of the present disclosure provides a method for preparing the above antibody-drug conjugate, which is selected from the following synthetic routes:
synthetic route 1:
   the compound of Formula (a) was condensed with HO-L^{z}-L^{j}' to afford the compound of Formula (b), and the compound of Formula (b) was conjugated with an antibody to afford the compound of Formula (I); wherein, R^{a} is -L¹-NH-L^{p}-Z-Ab, R^{b} is hydrogen; 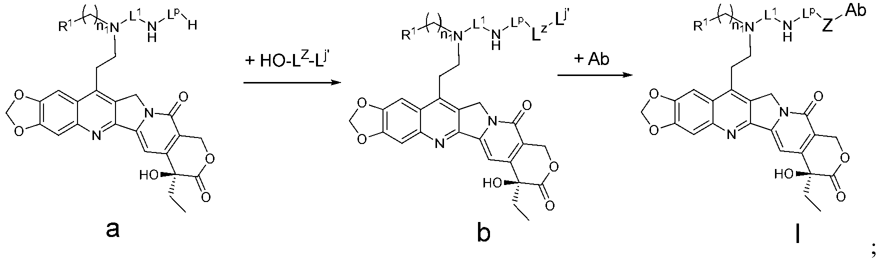
synthetic route 2:
   the compound of Formula (c) was condensed with HO-L^{z}-L^{j}' to afford the compound of Formula (d), and the compound of Formula (d) was conjugated with an antibody to afford the compound of Formula (I); wherein, R^{a} is hydrogen, R^{b} is selected from -L²-NH-L^{p}-Z-Ab; 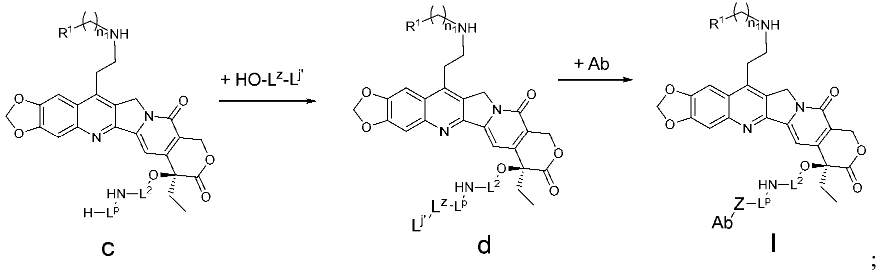
synthetic route 3:
   the compound of Formula (e) was condensed with HO-L^{z}-L^{j}' to afford the compound of Formula (f), and the compound of Formula (f)was conjugated with an antibody to afford the compound of Formula (I); wherein, R^{a} is hydrogen, R^{b} is selected from -L³-NH-Z-Ab; 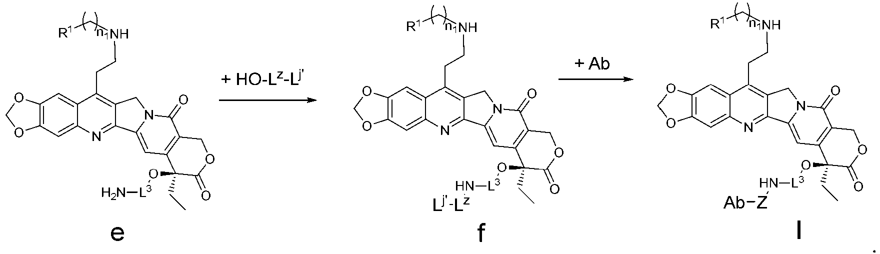
in the above synthetic routes, R¹, n₁, L¹, L², L³, L^{p}, L^{z}, Ab are each defined the same as the compound of Formula (I);
L^{j}' is selected from

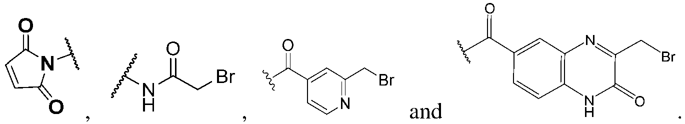

Another aspect of the present disclosure provides a pharmaceutical composition comprising the above antibody-drug conjugate or the antibody-drug conjugate prepared by the above method and a pharmaceutically acceptable carrier.

Another aspect of the present disclosure provides a use of the above antibody-drug conjugate, the antibody-drug conjugate prepared by the above method, or the above pharmaceutical composition in the manufacture of an anti-tumor medicament.

Another aspect of the present disclosure provides a method for preventing or treating a tumor disease in a patient in need thereof, comprising administering to the patient the above antibody-drug conjugate or pharmaceutical composition.

In some specific embodiments, the dosage of the above antibody-drug conjugate or pharmaceutical composition is a therapeutically effective amount.

In some specific embodiments, the tumor is selected from a solid tumor.

In some specific embodiments, the tumor is selected from esophageal cancer, lung cancer, non-small cell lung cancer, esophageal squamous cell carcinoma, ovarian cancer, lung cancer or breast cancer.

In some specific embodiments, the tumor non-small cell lung cancer.

### Description of Drawings

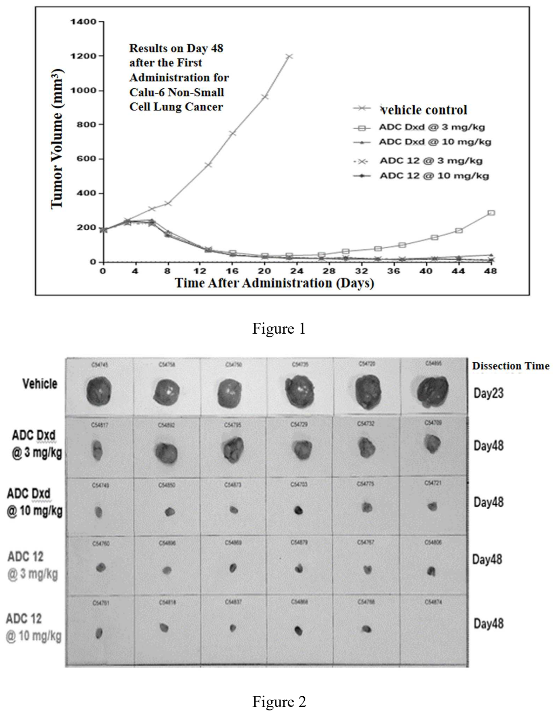

Figure 1 is a graph of the tumor growth curve for Calu-6 human non-small cell lung cancer CDX model in Assay 2.
Figure 2 is a photograph of tumors after dissection from the Calu-6 human non-small cell lung cancer CDX model in Assay 2.

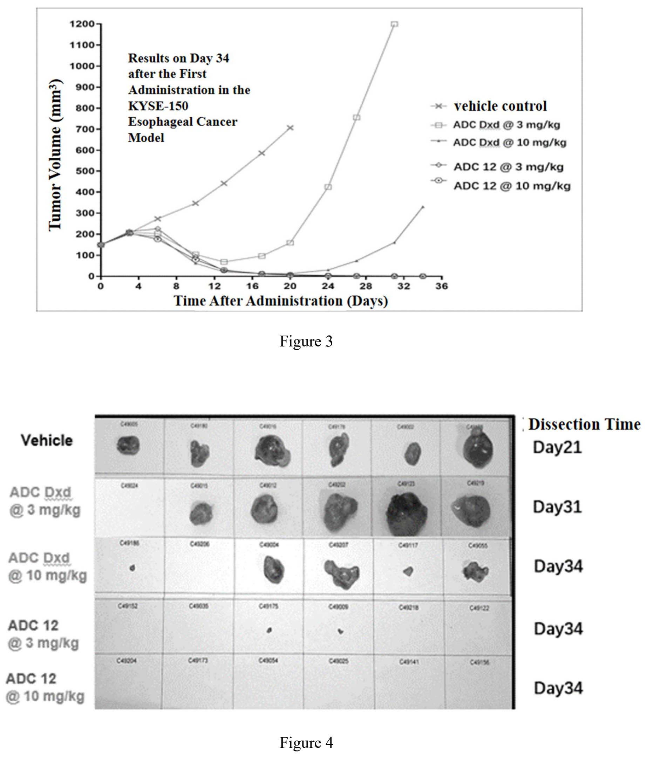

Figure 3 is a graph of the tumor growth curve for KYSE-150 human esophageal squamous cell carcinoma CDX model in Assay 2.
Figure 4 is a photograph of tumors after dissection from the KYSE-150 human esophageal squamous cell carcinoma CDX model in Assay 2.

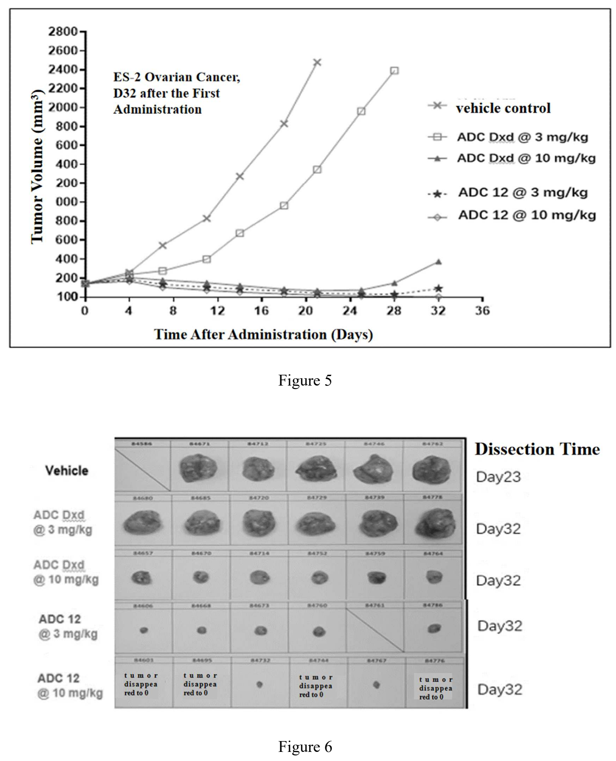

Figure 5 is a graph of the tumor growth curve for ES-2 human ovarian cancer CDX model in Assay 2.
Figure 6 is a photograph of tumors after dissection from the ES-2 human ovarian cancer CDX model in Assay 2.

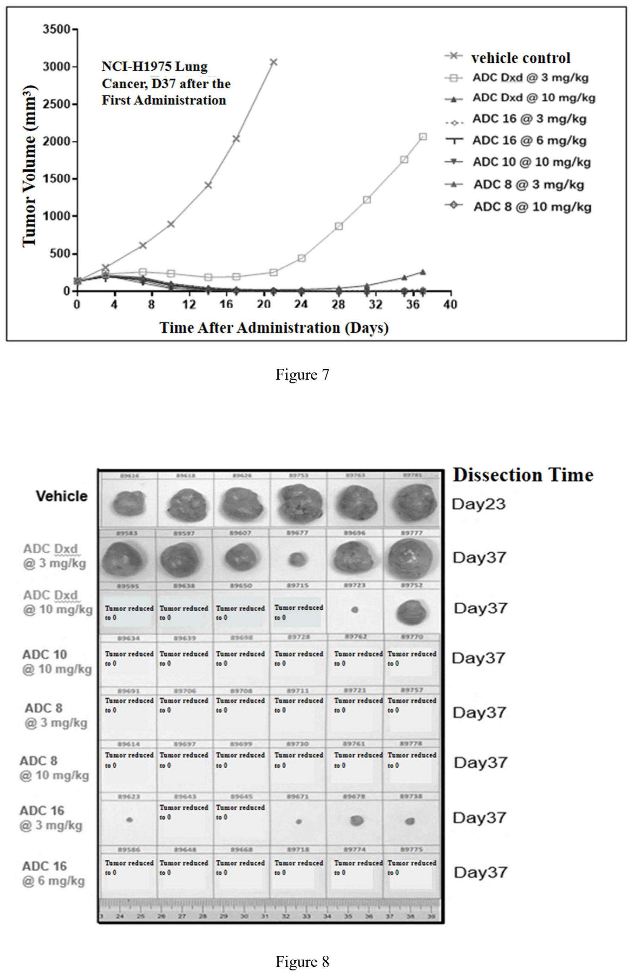

Figure 7 is a graph of the tumor growth curve for NCI-H1975 lung cancer CDX model in Assay 2.
Figure 8 is photograph of tumors after dissection from the NCI-H1975 lung cancer CDX model in Assay 2.

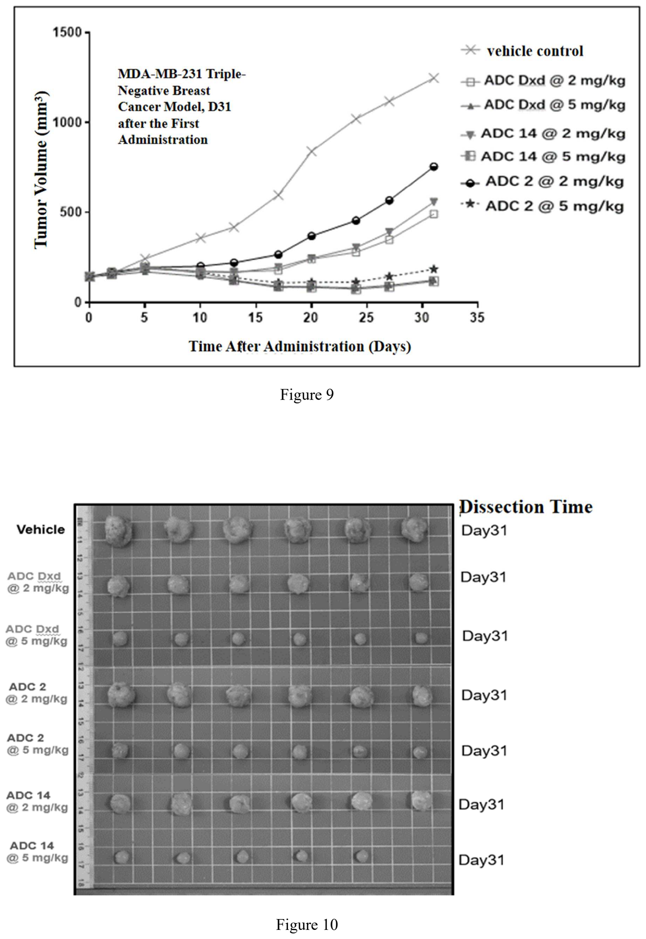

Figure 9 is a graph of the tumor growth curve for MDA-MB-231 triple-negative human breast cancer CDX model in Assay 2.
Figure 10 is a photograph of tumors after dissection from the MDA-MB-231 triple-negative human breast cancer CDX model in Assay 2.

### DETAILED DESCRIPTION OF THE EMBODIMENTS

### I. Definition

In the present disclosure, unless otherwise specified, scientific and technical terms used herein have the meanings commonly understood by those skilled in the art. Also, the relevant terms and laboratory procedures used herein are terms and conventional procedures widely used in the corresponding fields. Meanwhile, for a better understanding of the present disclosure, definitions and explanations of related terms are provided below.

As used herein and unless otherwise specified, the terms "comprising", "including", "having", "containing", including grammatical equivalents thereof, should generally be understood to be openended and non-limiting, for example, not excluding other unrecited elements or steps.

The compound of the present disclosure can be asymmetric, for example, having one or more stereoisomers. Unless otherwise specified, all stereoisomers are included, for example, enantiomers and diastereomers. The stereoisomers include geometric isomers (such as cis and trans structures) and optical isomers (such as enantiomers), therapeutic substances consisting of monomers, racemates, racemic mixtures and pharmaceutically acceptable salts thereof. The compound of the present disclosure containing asymmetric carbon atoms can be isolated in optically active pure form or racemic form. Optically active pure forms can be resolved from racemic mixtures or synthesized by using chiral starting materials or chiral reagents. Racemates, diastereomers, enantiomers are all included within the scope of the present disclosure.

A numerical range as used herein refers to each integer within the given range. For example,"C1-C6" refers to the group that can have 1 carbon atom, 2 carbon atoms, 3 carbon atoms, 4 carbon atoms, 5 carbon atoms or 6 carbon atoms.

When any variable (such as Rn) appears more than once in the composition or structure of a compound, the definition thereof is independent in each case. Therefore, for example, if a group is substituted with 1-5 R's, then the group can optionally be substituted with up to 5 R's, and each R has independent options in every case. In addition, combinations of substituents and/or variants thereof are only allowed if such combinations result in the formation of stable compounds.

The term "C₁₋C₆ alkyl" refers to straight or branched alkyl groups having 1 to 6 carbon atoms. Specific examples of alkyl groups include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and various branched isomers thereof, etc.

The term "alkoxyl" refers to -O-(alkyl) and -O-(unsubstituted cycloalkyl), wherein the definition of alkyl is as described above. Non-limiting examples of alkoxyl include: methoxy, ethoxy, propoxy, butoxy, cyclopropoxy, cyclobutoxy, cyclopentoxy, cyclohexoxy. Alkoxyl can be optionally substituted or unsubstituted. When substituted, the substituents are preferably one or more of the following groups, which are independently selected from alkyl, alkenyl, alkynyl, alkoxyl, alkylthio, alkylamino, halogen, mercapto, cyano, nitro, chloro group, cycloalkyl, heterocycloalkyl, aryl, heteroaryl, cycloalkoxyl, heterocycloalkoxyl, cycloalkylthio, heterocycloalkylthio, carboxyl, or carboxylate.

The term "acyl" means -C(=O)R, where R is a substituted or unsubstituted alkyl, a substituted or unsubstituted cycloalkyl, or a substituted or unsubstituted heteroalkyl.

The term "sulfonyl" means -S(=O)₂R, where R is a substituted or unsubstituted alkyl, a substituted or unsubstituted cycloalkyl, or a substituted or unsubstituted heteroalkyl. 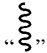 refers to the chemical bond junction. It should be noted that the structural fragments described in the disclosure (such as -L^{z}-L^{j}-), unless otherwise specified, represent the sequential linking of corresponding groups from left to right. For example, when -L¹²- is C₁-C₂ alkylene-O-, it means the left side of C₁-C₂ alkylene-O- is linked to -L¹¹-, and the right end is linked to -L¹³-.

Understandably, in the antibody-drug conjugates of the present disclosure, such as in formulas (I), (I-1), (I-2), (I-3), (I-4), (I-5), (I-6), the "-" between the drug-linker fragment and the antibody is intended to signify the linking relationship between the antibody and the fragment, and is not intended to limit the conjugate to one antibody linked to one drug-linker fragment. As is known in the art, due to the presence of multiple interchain disulfide bonds on an antibody, one antibody can be linked to one or more drugs.

### Medicament or pharmaceutical composition

The drugs or drug compositions of the present disclosure can be administered orally, topically, parenterally, or mucosally (e.g., sublingually, by inhalation, or rectally) in dose unit formulations comprising conventional non-toxic pharmaceutically acceptable carriers. An oral route is generally preferred. The active agents can be administered orally in the form of capsules, tablets, etc.

The term "treatment" includes inhibiting, alleviating, preventing, or eliminating one or more symptoms or side effects associated with the disease, condition, or disorder being treated.

The term "inhibition" is used in relation to controls. Those skilled in the art will readily determine the appropriate control for each experiment. For example, a reduced response in subjects or cells treated with the compound is compared to the response in subjects or cells not treated with the compound.

The term "patient" refers to an animal, preferably a mammal, and more preferably a human.

The term "pharmaceutical composition" refers to a composition that includes the compound or pharmaceutically acceptable salt thereof of the present disclosure, as well as at least one pharmaceutically acceptable component selected from the following, depending on the administration route and the nature of dosage form, including but not limited to: carriers, diluents, adjuvants, excipients, preservatives, fillers, disintegrants, wetting agents, emulsifiers, suspension agents, sweeteners, flavoring agents, fragrances, antimicrobials, antifungals, lubricants, dispersing agents, temperature-sensitive materials, temperature regulators, adhesives, stabilizers, and suspending agents.

The term "effective amount" or "therapeutically effective amount" refers to a sufficient amount of a drug or medication that is non-toxic but can achieve the desired effect. In embodiments of the present disclosure, when treating a patient in accordance with the present disclosure, the amount of drugs administered depends on many factors, such as the specific dosing regimen, the type of disease or condition and the severity thereof, the uniqueness (for example, body weight) of the treated person or host to be treated, but the amount of drug administered depends on specific surrounding circumstances, including, for example, the specific drug that has been employed, the route of administration, the treated condition, and the treated person or host to be treated, the administered dose may be routinely determined by methods known in the art. Generally, regarding the dosage used for adult therapy, the administered dose is typically within the range of 0.02-5000mg/day, for example, the range of about 1-1500mg/day. The required dose can be conveniently administered as a single dose, or as doses given simultaneously (or in a short period of time) or in divided doses at appropriate intervals, such as two, three, four doses per day or more. It can be understood by those skilled in the art that, although the above dose ranges are provided, the specific effective amount can be appropriately adjusted based on the patient's condition and in conjunction with a physician's diagnosis.

As used herein, the term "antibody-conjugate drug" has the same meaning as antibody-drug conjugate (ADC).

The term "Drug Antibody Ratio (DAR)" refers to the average number of payload molecules (antitumor compounds or drugs) attached to a single monoclonal antibody.

### Abbreviations:

### Fmoc: 9-fluorenylmethoxycarbonyl;

Amino acids constituting the peptide residue L^{P}: Val: valine, whose structural formula is 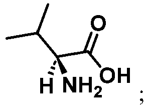 Ala: alanine, whose structural formula is 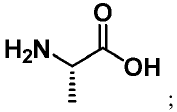 Gly: glycine, whose structural formula is 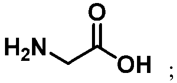 Phe: phenylalanine, whose structural formula is

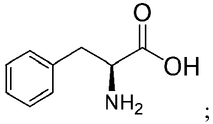

L^{P} can be obtained by amino acid condensation methods known in the art. PABC: 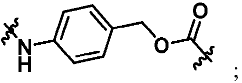 ha: 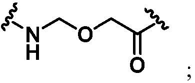 m:

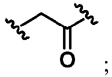

In the present disclosure, the antibody and the linker L^{j} for connecting to the antibody can be linked by methods known in the art. For example, when the structure of L^{j} is 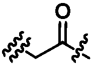 (or 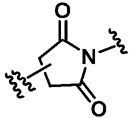 ), it can be linked to a reactive group such as a thiol on the antibody through 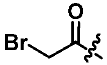 ( or 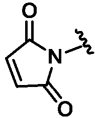 ) to afford, which is not particularly limited in the present disclosure.

### II. Example

In the example, the preparation of ADC uses, but not limited to, pertuzumab and IP140B antibodies. The heavy chain amino acid sequence of pertuzumab (HS627, PERTUZUMAB) is as follows (SEQ ID NO: 1):

EVQLVESGGGLVQPGGSLRLSCAASGFTFTDYTMDWVRQAPGKGLEWVADVNPNSGGSIYN
QRFKGRFTLSVDRSKNTLYLQMNSLRAEDTAVYYCARNLGPSFYFDYWGQGTLVTVSSASTK
GPSVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSREEMTKNQVSLTCLVKGFY
PSDIAVEWESNGQPENNYKTTPPVLDSDGSFFLYSKLTVDKSRWQQGNVFSCSVMHEALHNH
YTQKSLSLSPG

The light chain amino acid sequence is as follows (SEQ ID NO: 2):

DIQMTQSPSSLSASVGDRVTITCKASQDVSIGVAWYQQKPGKAPKLLIYSASYRYTGVPSRFSG
SGSGTDFTLTISSLQPEDFATYYCQQYYIYPYTFGQGTKVEIKRTVAAPSVFIFPPSDEQLKSGTA
SVVCLLNNFYPREAKVQWKVDNALQSGNSQESVTEQDSKDSTYSLSSTLTLSKADYEKHKV
YACEVTHQGLSSPVTKSFNRGEC

The heavy chain amino acid sequence of the IP140B antibody is as follows (SEQ ID NO: 3):

QVQLVQSGGGLVQPGGSLRLSCAASGFTFSSYEMNWVRQAPGKGLEWVSYISSSGSTIYYADS
VKGRFTISRDNAKNSLYLQMNSLRAEDTAVYYCAREMQFGWELLGAFDIWGQGTMVTVSSA
STKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKRVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEAL
HNHYTQKSLSLSPGK

The light chain amino acid sequence is as follows (SEQ ID NO: 4):

EIVLTQSPGTLSLSPGERATLSCRASQSVSSSYLAWYQQKPGQAPRLLIYGASSRATGIPDRFSG
SGSGTDFTLTISRLEPEDFAVYYCQQYGSSFGQGTKLEIKRTVAAPSVFIFPPSDEQLKSGTASVV
CLLNNFYPREAKVQWKVDNALQSGNSQESVTEQDSKDSTYSLSSTLTLSKADYEKHKVYACE
VTHQGLSSPVTKSFNRGEC.

Unless otherwise specified, the raw materials and equipment used in the specific embodiments of the present disclosure are all known products, obtained by purchasing commercially available products.

### Intermediate HX-16a: 7-(N-(2',2'-difluoroethyl)amino)ethyl-10,11-methylenedioxycamptothecin

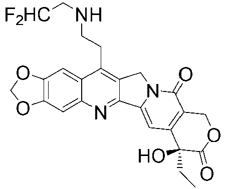

To a reaction flask, difluoroethylamine (1.0 g, 12 mmol), hydrochloric acid (1.4 mL, 16 mmol), 7-methyl-10,11-methylenedioxycamptothecin (1.0 g, 2.46 mmol), and DMSO (5 mL) were added. The mixture was stirred and heated to 120°C, and reacted for 1 hour, then cooled. Isopropanol was added, and the mixture was filtered and purified by silica gel column chromatography to afford the title compound. (393 mg, yield 32%, HPLC 95%); ¹H NMR (500 MHz, DMSO-d₆) δ 7.68 (s, 1H), 7.52 (s, 1H), 7.28 (s, 1H), 6.58 (s, 1H), 6.33 (s, 2H), 6.02 (t, J=56.6 Hz, 1H), 5.47 (s, 2H), 5.26 (s, 2H), 3.26 (s, 3H), 2.94 (s, 4H), 1.92 (s, 2H), 0.93 (s, 3H); LC-MS (M+H)⁺ 500.13 (theoretical value 499.16).

### Intermediate HX-22e: 7(N-(2',2',2'-trifluoroethyl)amino)ethyl-10,11-methylenedioxycamptothecin

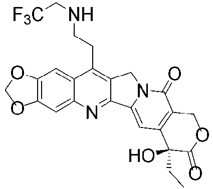

Trifluoroethylamine hydrochloride (100 mg, 0.74 mmol) and 7-methyl-10,11-methylenedioxycamptothecin (100 mg, 0.25 mmol) were dissolved in DMSO (3 mL). The mixture was stirred and heated to 120 °C for 1 hour. After cooling, methyl tert-butyl ether was added, and the mixture was filtered and purified by silica gel column chromatography to afford the title compound. (49mg, yield 38%, HPLC 99%); ¹H NMR (500 MHz, DMSO-d₆) δ 7.71 (s, 1H), 7.54 (s, 1H), 7.29 (s, 1H), 6.55 (s, 1H), 6.34 (d, J=2.0 Hz, 2H), 5.48 (d, *J*=3.0 Hz, 2H), 5.30 (s, 2H), 3.42 (s, 2H), 3.42 (s, 2H), 3.29 (s, 2H), 1.92 (dd, J=14.4, 7.3 Hz, 2H), 0.94 (t, J=7.3 Hz, 4H); LC-MS (M+H)⁺ 518.34 (theoretical value 517.15).

### Example 1: 7-(N-(HS627-Ac-PEG₂-Gly-Gly-Leu-Gly-ha-Ac), N-(2',2'-difluoroethyl)amino)ethyl-10,11-methylenedioxycamptothecin (1)

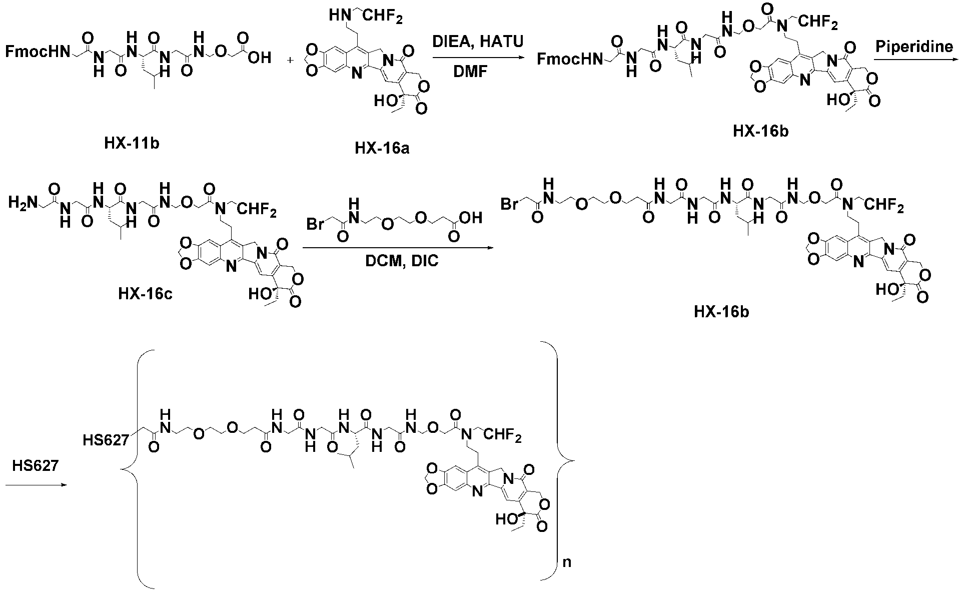

To a reaction flask, **HX-11b** (0.1 g, 0.16 mmol), 3 mL of DMF, HATU (74.6 mg, 0.2 mmol), DIPEA (25.3 mg, 0.2 mmol), and **HX-16a** (82 mg, 0.16 mmol) were added. The mixture was reacted at room temperature for 1.5 h, then purified by silica gel column chromatography to afford **HX-16b** as a yellow solid (153 mg, yield 85%); LCMS: (M+1)⁺ 1093.11 (theoretical value: 1092.40).

In a 10 mL reaction vial, **HX-16b** (153 mg, 0.14 mmol) and piperidine (140 mg, 1.6 mmol) were added in sequence. The mixture was stirred to react at room temperature for 1 h. To the reaction solution, 30 mL of methyl tert-butyl ether was added. The mixture was centrifuged (5 min, 10000r/min), and the supernatant was discarded, 1 mL of methanol was added again, and the mixture was sonicated until clear. Then, 30 mL of methyl tert-butyl ether was added again. The mixture was centrifuged (5 min, 10000r/min), and the supernatant was removed. The solvent was removed under reduced pressure to afford **HX-16c** as a solid (103.5 mg, yield 72.7%). LCMS: (M+1)⁺871.21 (theoretical value: 870.34), which was used directly in the next step without further purification.

In a 50 mL single-neck flask, DCM (20 mL), 3-(2-(2-aminoethoxy)ethoxy)propionic acid (2 g, 11.3 mmol), and bromoacetyl bromide (2.27 g, 11.3 mmol) were added and the mixture was stirred for 30 min, and then 10 mL of saturated sodium chloride was added to the reaction solution for extraction. The DCM phase was dried over anhydrous magnesium sulfate to afford the intermediate **HX-3b** (3.1 g, yield 89%). LCMS showed (M+1)⁺298.00 (theoretical value: 297.02).

In a 10 mL single-neck flask, DCM (3mL), **HX-16c** (60 mg, 0.069 mmol), **HX-3b** (25 mg, 0.083 mmol) and DIC (10.5 mg, 0.083 mmol) were added in sequence. After stirring to react at room temperature for 30min, the reaction solution was injected into a 25 g C18 pre-column (pre-equilibrated with acetonitrile, then with water, the water phase contains 0.1%HCOOH), then eluted via medium-pressure reverse-phase C18 chromatography (gradient: 5% to 40% acetonitrile in water over 30min). The product was lyophilized to afford **HX-16** as a yellow solid (24.5 mg, yield 30.9%); LCMS: (M+1)⁺ 1150.35 (theoretical value: 1149.35).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-16**(0.92 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 1** (3 mg/mL, 2 mL).

UV-HPLC calculated average value: n=6.8.

### Example 2: 7-(N-(IP140B-Ac-PEG₂-Gly-Gly-Leu-Gly-ha-Ac), N-(2',2'-difluoroethyl)amino)ethyl-10,11-methylenedioxycamptothecin (2)

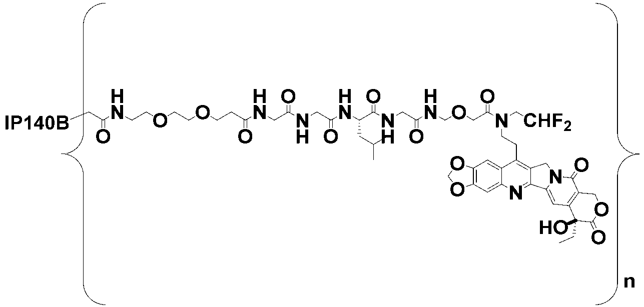

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

**Compound HX-16** (0.92 mg, 0.8 mmol) was dissolved in 0.09 mL DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 2** (3.2mg/mL, 2 mL).

UV-HPLC calculated average value: n=7.0.

### Example 3: 7-(N-(HS627-Ac-PEG₂-Gly-Val-Ala-Gly-ha-Ac), N-(2',2'-difluoroethyl)amino)ethyl-10,11-methylenedioxycamptothecin (3)

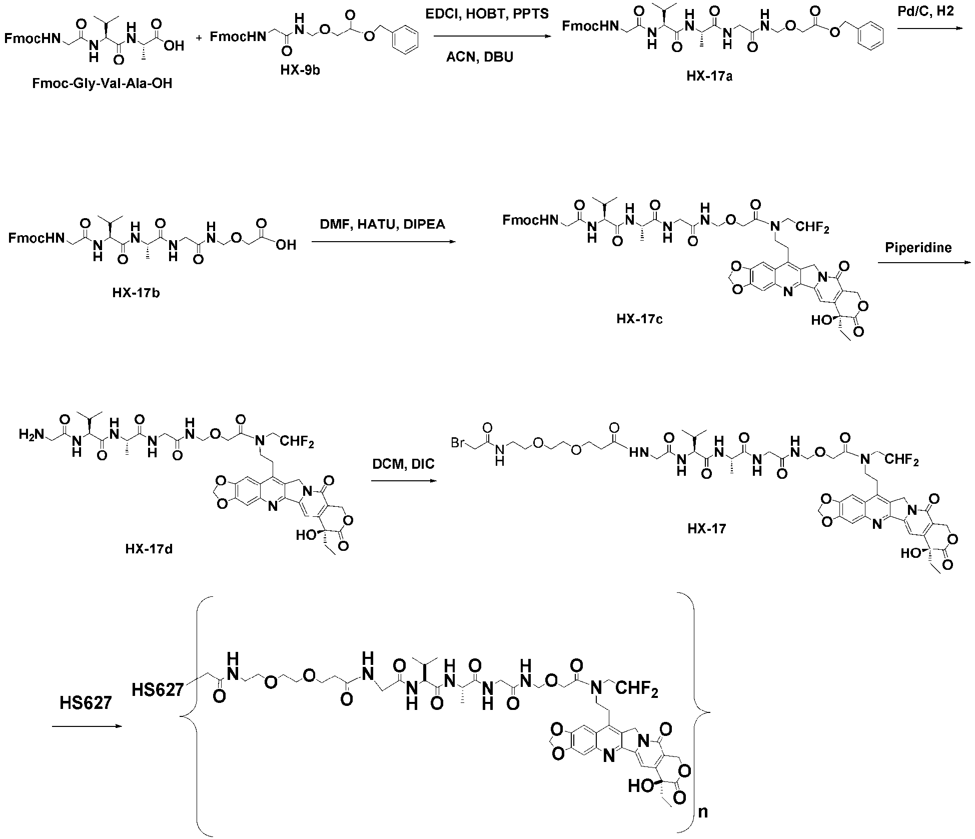

**HX-9b** (3 g, 6.32 mmol) was taken and 100 mL of acetonitrile was added. Then DBU (0.48 g, 3.16 mmol) was added. The mixture was reacted at room temperature for 2 h. PPTS (0.79 g, 3.16 mmol), HOBT (0.85 g, 6.32 mmol), Fmoc-Gly-Val-Ala-OH (2.61 g, 5.36 mmol), and EDCI (1.21 g, 6.32 mmol) were added in sequence. After the addition was completed, the mixture was reacted at room temperature for 15 h. The mixture was evaporated under reduced pressure, and purified by silica gel column chromatography to afford **HX-17a** as a white powdery solid (2.75 g, yield 62%); LCMS: (M+1)⁺ 702.15 (theoretical value: 701.31)

In a 500 mL single-necked flask, **HX-17a** (2.75 g, 3.9 mmol) was dissolved in a mixed solvent of 50 mL of methanol and 25 mL of DCM. 1 g of 10% palladium on carbon was added. After purging with hydrogen gas, the mixture was reacted at room temperature under atmospheric pressure for 2 h. After the reaction was completed, the mixture was filtered and concentrated to afford the product **HX-17b** (2.2 g, yield 92% ); LCMS: (M+1)⁺612.18 (theoretical value: 611.26).

**HX-16a** (50 mg, 0.1 mmol) was taken and 2 mL of DMF, HATU (38 mg, 0.1 mmol), DIPEA (26 mg, 0.2 mmol), and **HX-17b** (61 mg, 0.1 mmol) were added. The mixture was reacted at room temperature for 15 h. 200 mL of DCM was added for dilution, the mixture was washed with saturated sodium chloride solution, dried over anhydrous sodium sulfate, and purified by silica gel column chromatography to afford **HX-17c** as a yellow solid (85 mg, yield 78%); LCMS: (M+1)⁺ 1093.11 (theoretical value: 1092.40).

**HX-17c** (78 mg, 0.07 mmol) was dissolved in 0.2 mL of DMF, then piperidine (55 mg, 0.7 mmol) was added. The mixture was stirred to react at room temperature for 1 h. Then, 30 mL of methyl tert-butyl ether was added to the reaction solution. The mixture was centrifuged (5 min, 10,000 r/min), and the supernatant was removed, and the solvent was evaporated under reduced pressure to afford **HX-17d** as a solid (56 mg, yield 73.4%); LCMS: (M+1)⁺ 871.32; calculated value: 870.34). The intermediate was used directly in the next step without further purification.

In a 10 mL single-neck flask, DCM (3mL), **HX-17d** (56 mg, 0.064 mmol), **HX-3b** (18.6 mg, 0.064 mmol) and DIC (8.1 mg, 0.064 mmol) were added in sequence. The mixture was stirred to react at room temperature for 90 min. Then reaction solution was injected into a 25 g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30min). The product was lyophilized to afford **HX-17** as a yellow solid (23mg, yield 31%); LCMS: (M+1)⁺ 1150.33 (theoretical value: 1149.35).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-17** (0.92 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 3** (2.9 mg/mL, 2 mL).

UV-HPLC calculated average value: n=7.4.

### Example 4: 7-(N-(IP140B-Ac-PEG₂-Gly-Val-Ala-Gly-ha-Ac), N-(2',2'-difluoroethyl)amino)ethyl-10,11-methylenedioxycamptothecin (4)

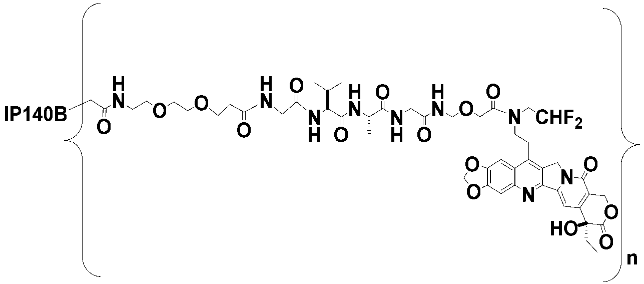

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-17**(0.92 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 4** (3mg/mL, 2 mL).

UV-HPLC calculated average value: n=7.5.

### Example 5: 7-(N-(HS627-Ac-PEG₂-Gly-Gly-Phe-Gly-m), N-(2',2'-difluoroethyl)amino)ethyl-10,11-methylenedioxycamptothecin (5)

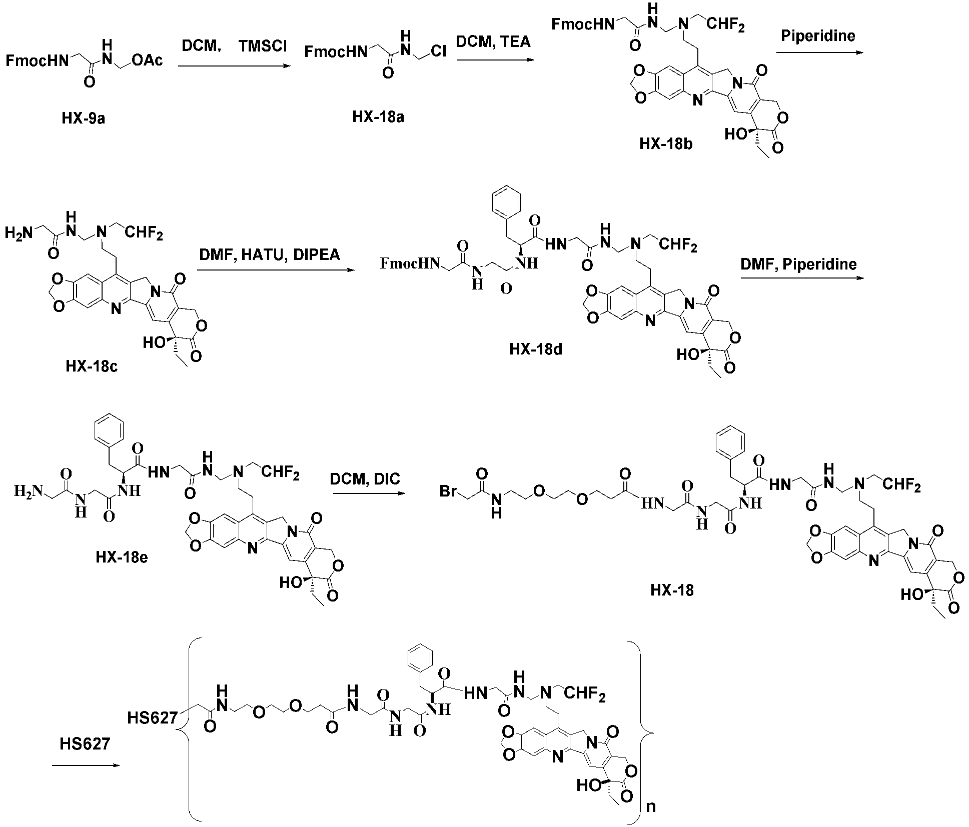

In a 100 mL three-necked flask, HX-9a (1 g, 2.7 mmol) was dissolved in 10 mL of DCM. Then 8 mL of TMSCl was added, and the reaction system was protected with argon. The mixture was stirred at room temperature for 2 hours and concentrated under reduced pressure to afford HX-18a as a white solid, which was directly used in the next step; LCMS: (M+1)⁺ 344.80 (theoretical value: 344.09).

HX-16a (100 mg, 0.2 mmol) was dissolved in 5 mL of anhydrous DCM. HX-18a (0.93 g, 2.7 mmol) was added, and the reaction solution was reacted at room temperature for 2 hours. Water (10 mL) was added, and the mixture was extracted with DCM (20 mL*3) three times. The organic phase was directly concentrated and purified by silica gel column chromatography to afford compound HX-18b (121 mg, yield 75%); LCMS: (M+1)⁺ 808.13 (theoretical value: 807.28).

In a 10 mL single-neck flask, HX-18b (121 mg, 0.149 mmol) was added and dissolved with 1 mL of DMF. Then, 100 µL of piperidine was added, and the mixture was reacted at room temperature for 1 h. After the reaction was completed, the reaction solution was added dropwise to 20 mL of methyl tert-butyl ether. The mixture was centrifuged, and the supernatant was discarded, and the residue was dried under vacuum to afford HX-18c (68 mg, yield 78%); LCMS: (M+1)⁺586.21 (theoretical value: 585.20).

HX-18c (68 mg, 0.11 mmol) was weighed and dissolved in 2 mL of DMF. DIPEA (15 mg, 0.11 mmol) was added and stirred until clear. Fmoc-Gly-Gly-Phe-OH (56 mg, 0.11 mmol) and HATU (42 mg, 0.11 mmol) were added in sequence. The mixture was stirred at room temperature, purified by column chromatography to afford the product HX-18d (107 mg, yield 86%); LCMS: (M+1)⁺ 1069.09 (theoretical value: 1068.38).

In a 10 mL single-neck flask, HX-18d (107 mg, 0.1 mmol) was added and dissolved with 1 mL of DMF. Then 100 µL of piperidine was added, and the mixture was reacted under room temperature for 1 h. After the reaction was completed, the reaction solution was added dropwise to 20 mL of methyl tert-butyl ether. The mixture was centrifuged, and the supernatant was removed, and the residue was dried to afford HX-18e (56 mg, yield 66%); LCMS: (M+1)⁺ 847.23 (theoretical value: 846.31).

In a 10 mL single-neck flask, DCM (3 mL), HX-18e (56 mg, 0.066 mmol), **HX-3b** (19.2 mg, 0.066 mmol) and DIC (8.4 mg, 0.066 mmol) were added in sequence. The mixture was stirred to react at room temperature for 90 min, concentrated, and purified by silica gel column chromatography to afford the product **HX-18** as a yellow solid (12mg, yield 16%); LCMS: (M+1)⁺ 1126.24 (theoretical value: 1125.33).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-18** (0.90 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 5** (3 mg/mL, 2 mL).

UV-HPLC calculated average value: n=7.5.

### Example 6: 7-(N-(IP140B-Ac-PEG₂-Gly-Gly-Phe-Gly-m), N-(2',2'-difluoroethyl)amino)ethyl-10,11-methylenedioxycamptothecin (6)

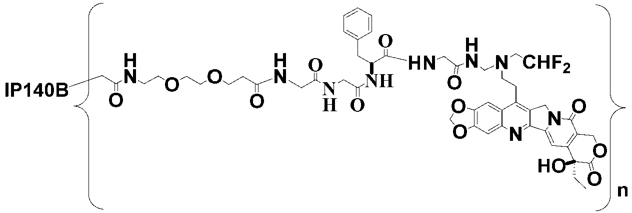

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-18** (0.90mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 6**(3.1 mg/mL, 2 mL).

UV-HPLC calculated average value: n=7.6.

### Example 7: 7-(N-(HS627-Ac-PEG₂-Gly-Lys-PABC), N-(2',2'-difluoroethyl)amino)ethyl-10,11-methylenedioxycamptothecin (7)

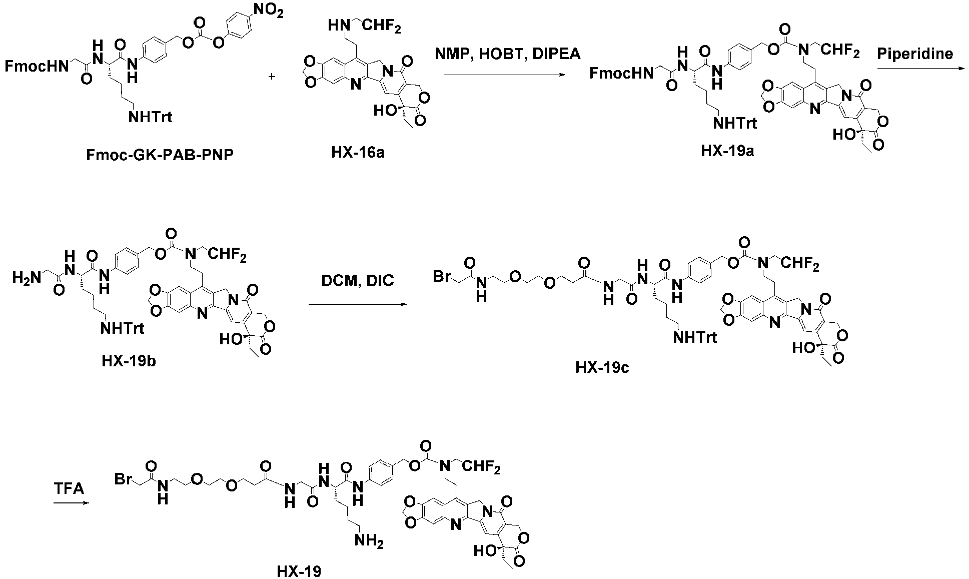

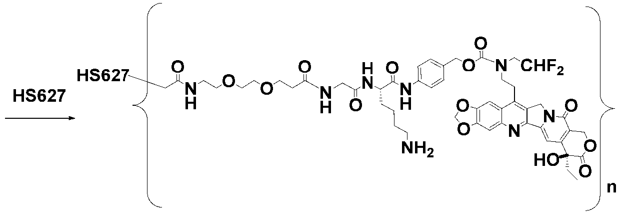

In a 10 mL single-neck flask, HX-16a (100 mg, 0.2 mmol), 1 mL of NMP, DIPEA (129 mg, 1 mmol), Fmoc-GK-PAB-PNP (187 mg, 0.2 mmol), and HOBt (27 mg, 0.2 mmol) were added in sequence. The mixture was stirred at room temperature for 4 h to afford HX-19a, which was directly used in the next step. LCMS: (M+1)⁺ 1298.41 (theoretical value: 1297.50).

In the above reaction solution of HX-19a, 0.1 mL of piperidine (V:V=10%) was added, and the mixture was reacted at room temperature for 0.5 h. Then 20 mL of methyl tert-butyl ether was added to the reaction solution. The mixture was centrifuged (5 min, 10,000 r/min), and the supernatant was removed, and the solvent was evaporated under reduced pressure to afford HX-19b as a solid(155 mg, yield 72%); LCMS: (M+1)⁺ 1076.39 (theoretical value: 1075.43). The intermediate was used directly in the next step without further purification.

In a 10 mL single-neck flask, DCM (2 mL), **HX-19b** (50 mg, 0.046 mmol), **HX-3b** (13.4 mg, 0.046 mmol), and DIC (5.85 mg, 0.046 mmol) were added in sequence. The mixture was stirred to react at room temperature for 90 min, followed by the addition of 0.1 mL of TFA and continued to react for 30 min. The reaction solution was injected into a a 25 g C18 pre-column (pre-equilibrated with acetonitrile, then with water, the water phase contains 0.1% TFA), followed by elution via medium-pressure reverse-phase C18 chromatography (gradient: 5% to 40% acetonitrile in water over 30 min). The product was lyophilized to afford **HX-19** as a yellow solid (21 mg, yield 40%); LCMS: (M+1)⁺ 1113.24 (theoretical value: 1112.33).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-19** (0.89 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 7**(3.0mg/mL, 2 mL).

UV-HPLC calculated average value: n=7.1.

### Example 8: 7-(N-(IP140B-Ac-PEG₂-Gly-Lys-PABC), N-(2',2'-difluoroethyl)amino)ethyl-10,11-methylenedioxycamptothecin (8)

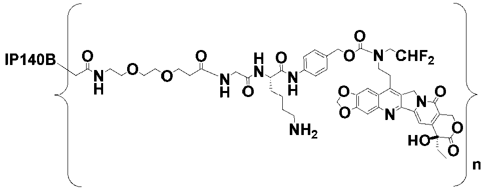

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-19** (0.89mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 8**(3.3mg/mL, 2 mL).

UV-HPLC calculated average value: n=7.2.

### Example 9: 7-(N-(HS627-isonicotinoyl-PEG₂-Gly-Lys-PABC), N-(2',2'-difluoroethyl)amino)ethyl-10,11-methylenedioxycamptothecin (9)

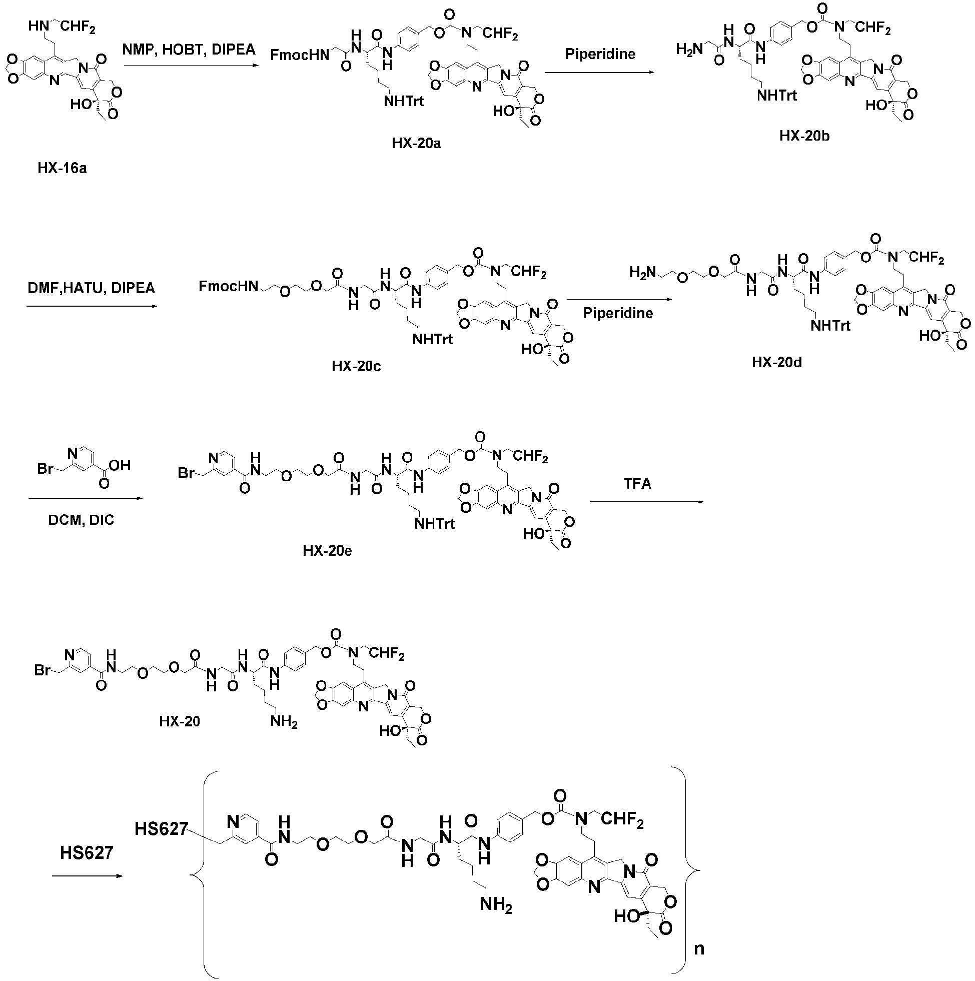

In a 10 mL single-neck flask, HX-16a (100 mg, 0.2 mmol), 1 mL of NMP, DIPEA (129 mg, 1 mmol), Fmoc-GK-PAB-PNP (187 mg, 0.2 mmol), HOBt (27 mg, 0.2 mmol) were added in sequence. The mixture was stirred to react at room temperature for 4 h to afford HX-20a, LCMS: (M+1)⁺ 1298.41 (theoretical value: 1297.50),which was used directly in the next step.

In the above reaction solution of HX-20a, 0.1 mL of piperidine (V:V=10%) was added, and the mixture was reacted at room temperature for 0.5 h. Then 20 mL of methyl tert-butyl ether was added to the reaction solution. The mixture was centrifuged (5 min, 10,000 r/min), and the supernatant was removed, and the solvent was evaporated under reduced pressure to afford HX-20b as a solid (155 mg, yield 72%); LCMS: (M+1)⁺ 1076.42 (theoretical value: 1075.43), which was directly used in the next step.

HX-20b (155 mg, 0.14 mmol) was weighed and dissolved in 2 mL of DMF. DIPEA (18.6 mg, 0.14 mmol) was added and the mixture was stirred until clear. Fmoc-NH-PEG2-CH₂CH₂-COOH (57 mg, 0.14 mmol) and HATU (55 mg, 0.14 mmol) were added in sequence. The mixture was stirred to react at room temperature, then purified by column chromatography to afford the product HX-20c as a solid (168 mg, yield 81%); LCMS: (M+1)⁺ 1443.57 (theoretical value: 1442.57).

In a 10 mL single-neck flask, HX-20c (168 mg, 0.11 mmol) was added and dissolved with 1 mL of DMF. Then, 100 µL of piperidine was added, and the mixture was reacted at room temperature for 1 h. After the reaction was completed, the reaction solution was added dropwise to 20 mL of methyl tert-butyl ether. The mixture was centrifuged, and the supernatant was removed, and the residue was dried to afford the product HX-20d as a solid (126 mg, yield 88%); LCMS: (M+1)⁺ 1221.31 (theoretical value: 1220.50).

In a 10 mL single-neck flask, DCM (2 mL), **HX-20d** (126 mg, 0.10 mmol), 2-(bromomethyl)isonicotinic acid (22 mg, 0.10 mmol) and DIC (13 mg, 0.10 mmol) were added in sequence. The mixture was stirred to react at room temperature for 90 min, followed by the addition of 0.1 mL of TFA and continued to react for 30 min. The reaction solution was injected into a 25 g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30min). The product was lyophilized to afford **HX-20** as a yellow solid (32 mg, yield 25%); LCMS: (M+1)⁺ 1176.28 (theoretical value: 1175.34).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-20** (0.94mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate (3.1mg/mL, 2 mL).

UV-HPLC calculated average value: n=7.3

### Example 10: 7-(N-(IP140B-isonicotinoyl-PEG₂-Gly-Lys-PABC), N-(2',2'-difluoroethyl)amino)ethyl-10,11-methylenedioxycamptothecin (10)

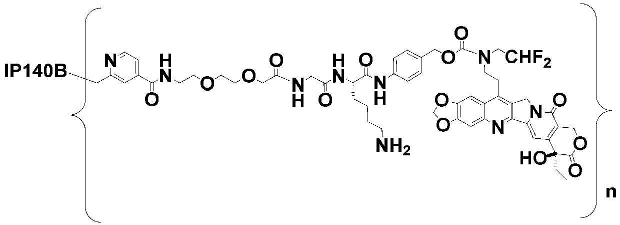

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-20** (0.94mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 10** (3.4mg/mL, 2 mL).

UV-HPLC calculated average value: n=7.2.

### Example 11: 7-(N-(HS627-Ac-PEG₂-Val-Ala-PABC), N-(2',2'-difluoroethyl)amino)ethyl-10,11-methylenedioxycamptothecin (11)

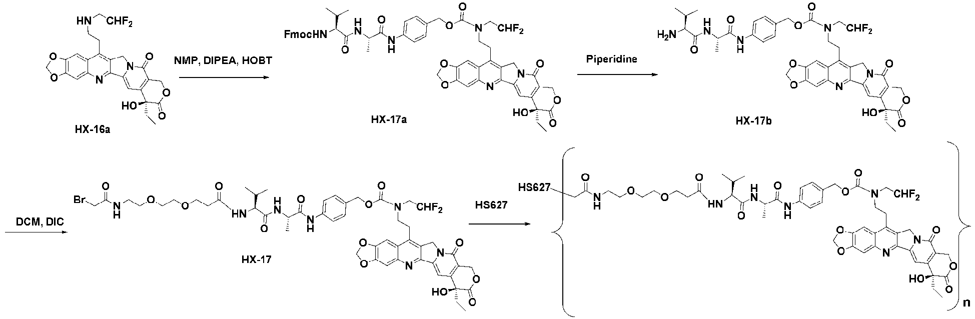

In a 10 mL single-neck flask, HX-16a (100 mg, 0.20 mmol), 0.5 mL of NMP, DIPEA (130 mg, 1 mmol), Fmoc-VA-PAB-PNP (138 mg, 0.20 mmol), HOBt (27.4 mg, 0.20 mmol) were added in sequence. The mixture was stirred to react at room temperature for 4h to afford **HX-21a,** which was directly used in the next step without further purification; LCMS: (M+1)⁺ 1041.07 (theoretical value: 1040.38).

In the above reaction solution of HX-21a, 0.5 mL of piperidine (V:V=10%) was added, and the mixture was reacted at room temperature for 30 min. Then 20 mL of methyl tert-butyl ether was added to the reaction solution. The mixture was centrifuged (5 min, 10,000 r/min), and the supernatant was removed, and the solvent was evaporated under reduced pressure to afford the product **HX-21b** as a solid (123 mg, yield 75%); LCMS: (M+1)⁺ 819.22 (theoretical value: 818.31).

In a 10 mL single-neck flask, DCM (1mL), **HX-17b** (123 mg, 0.15 mmol), **HX-3b** (43.45 mg, 0.15 mmol) and DIC (18.9 mg, 0.15 mmol) were added in sequence. The mixture was stirred to react at room temperature for 90 min. The reaction solution was injected into a 25 g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30 min). The product was lyophilized to afford **HX-21** as a yellow solid (44 mg, yield 26%); LCMS: (M+1)⁺ 1098.21 (theoretical value: 1097.32).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-21** (0.88 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 11**(3.2mg/mL, 2 mL).

UV-HPLC calculated average value: n=7.5.

### Example 12: 7-(N-(IP140B-Ac-PEG₂-Val-Ala-PABC), N-(2',2'-difluoroethyl)amino)ethyl-10,11-methylenedioxycamptothecin (12)

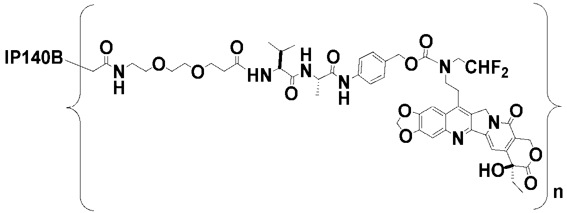

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-21** (0.88 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 12** (3.1 mg/mL, 2 mL).

UV-HPLC calculated average value: n=7.6.

### Example 13: 7-(N-(HS627-Ac-PEG₂-Gly-Leu-Gly-m), N-(2',2',2'-trifluoroethyl)amino)ethyl-10,11-methylenedioxycamptothecin (13)

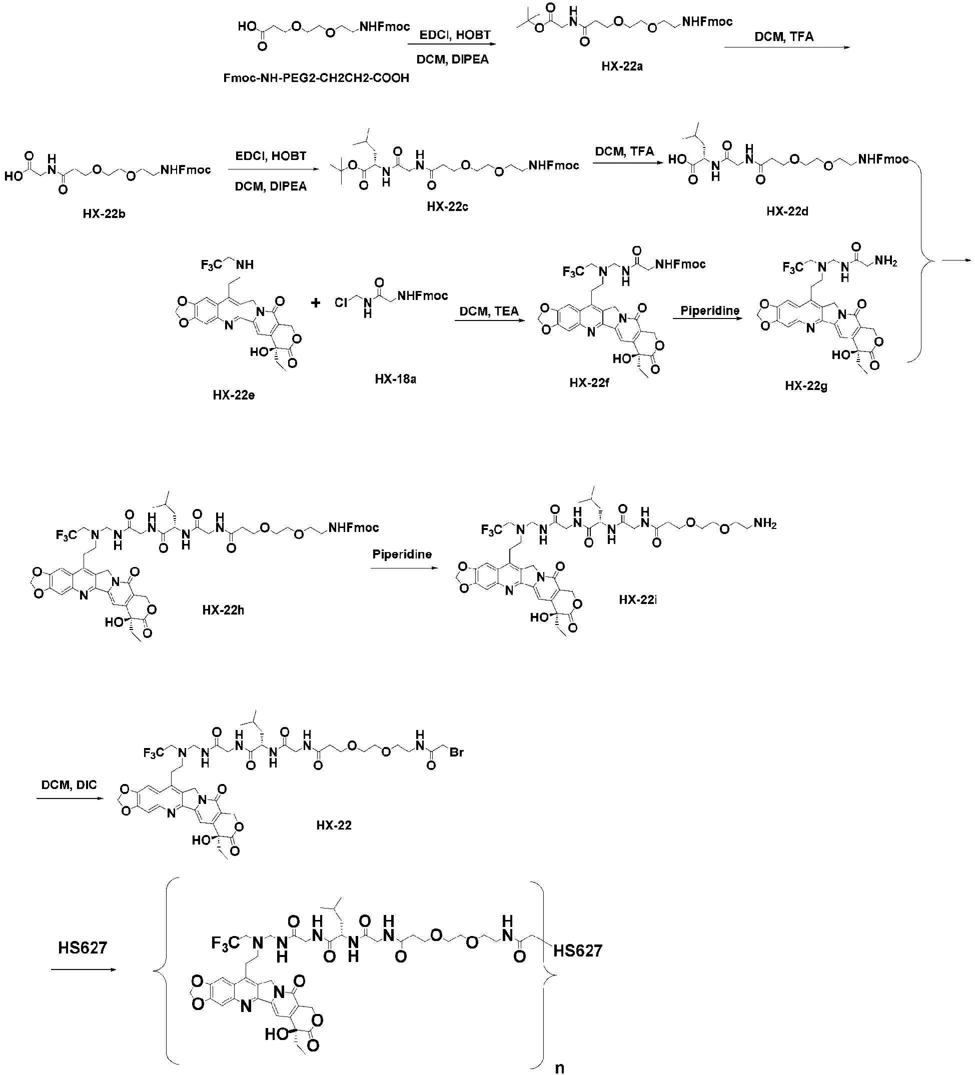

In a 1000 mL single-neck flask, 500 mL of dichloromethane was added. Under stirring, Fmoc-NH-PEG₂-CH₂CH₂-COOH (30 g, 75 mmol), HOBT (12.15 g, 90 mmol), EDCI (17.25 g, 90 mmol), glycine tert-butyl ester (10.32 g, 78.75 mmol), and DIPEA (10.16 g, 90 mmol) were added in sequence. The mixture was stirred at room temperature overnight. The reaction solution was washed with saturated sodium chloride (300mL*3), and the organic phase was extracted with saturated sodium bicarbonate (300mL*3) and 0.5 M hydrochloric acid (300mL*3) in sequence. After phase separation, the DCM phase was dried over anhydrous magnesium sulfate, filtered to remove anhydrous magnesium sulfate, and concentrated to afford the product **HX-22a** (36.2 g, yield 94%); LCMS: (M+1)⁺ 513.14 (theoretical value: 512.25).

In a 1000 mL single-neck flask, 400 mL of dichloromethane was added, then **HX-22a** (36.2 g, 70 mmol) was added. After stirring uniformly, 100 mL of trifluoroacetic acid was added dropwise. The mixture was stirred at room temperature overnight and concentrated to afford the product **HX-22b** (32.2 g, yield 100%); LCMS: (M+1)⁺457.08 (theoretical value: 456.19).

In a 1000 mL single-neck flask, 300 mL of dichloromethane was added. Under stirring, **HX-22b** (32.2 g, 70 mmol), HOBT (11.34 g, 84 mmol), EDCI (16.10 g, 84 mmol), glycine tert-butyl ester (9.62 g, 73.5 mmol), and DIPEA (10.84 g, 84 mmol) were added in sequence. The mixture was stirred at room temperature overnight. The reaction solution was washed with saturated sodium chloride (150mL*3), and the organic phase was extracted with saturated sodium bicarbonate (150mL*3) and 0.5 M hydrochloric acid (150mL*3) in sequence. After phase separation, the DCM phase was dried over anhydrous magnesium sulfate, filtered to remove anhydrous magnesium sulfate, and concentrated to afford the product **HX-22c** (42 g, yield 95%); LCMS: (M+1)⁺ 626.18 (theoretical value: 625.34).

In a 1000 mL single-neck flask, 400 mL of dichloromethane was added, then **HX-22c** (42 g, 67.2 mmol) was added. After stirring uniformly, 100 mL of trifluoroacetic acid was added dropwise. The mixture was stirred at room temperature overnight and concentrated to afford the product **HX-22d** (38.23 g, yield 100%); LCMS: (M+1)⁺ 570.14 (theoretical value: 569.27).

**HX-22e** (100 mg, 0.19 mmol) was dissolved in 5 mL of anhydrous DCM. **HX-18a** (665.3 mg, 1.9 mmol) was added, and the reaction solution was stirred at room temperature for 2 hours, concentrated, and purified by silica gel column chromatography to afford compound **HX-22f** (47 mg, yield 30%); LCMS: (M+1)⁺ 826.19 (theoretical value: 825.26).

In a 10 mL single-neck flask, **HX-22f** (47 mg, 0.056 mmol) was added and dissolved in 1 mL of DMF. Then, 100 µL of piperidine was added, and the reaction was reacted at room temperature for 1 h. After the reaction was completed, the reaction solution was added dropwise to 20 mL of methyl tert-butyl ether, centrifuged, the supernatant was removed, and the residue was dried to afford **HX-22g** (21 mg, yield 60%); LCMS: (M+1)⁺ 604.03 (theoretical value: 603.19).

**HX-22g** (21 mg, 0.034 mmol) was weighed and dissolved in 2 mL of DMF. DIPEA (4.49 mg, 0.034 mmol) was added, and the mixture was stirred uniformly. **HX-22d** (19.8 mg, 0.034 mmol) and HATU (13.23 mg, 0.034 mmol) were added in sequence The mixture was stirred at room temperature for 1 h, concentrated, and purified by silica gel column chromatography to afford compound **HX-22h** (35 mg, yield 87.5%); LCMS: (M+1)⁺ 1155.22 (theoretical value: 1154.46).

In a 10 mL single-neck flask, **HX-22h** (35 mg, 0.03 mmol) was added and dissolved in 1 mL of DMF. Then, 100 µL of piperidine was added, and the mixture was reacted at room temperature for 1 h. After completion, the reaction solution was added dropwise to 20 mL of methyl tert-butyl ether, centrifuged, the supernatant was removed, and the residue was dried to afford **HX-22i** (22 mg, yield 78.9%); LCMS: (M+1)⁺ 933.65 (theoretical value: 932.95).

In a 10 mL single-neck flask, DCM (3 mL), **HX-22i** (22 mg, 0.023 mmol), **HX-3b** (7 mg, 0.023 mmol), and DIC (2.97 mg, 0.023 mmol) were added in sequence. The mixture was stirred at room temperature for 90 min, concentrated, and purified by silica gel column chromatography to afford the product **HX-22** as a yellow solid (19 mg, yield 77%); LCMS: (M+1)⁺ 1053.27 (theoretical value: 1052.31).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-22** (0.84mg, 0.8mmol) was dissolved in 0.08 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 13** (3.3mg/mL, 2 mL).

UV-HPLC calculated average value: n=7.1.

### Example 14: 7-(N-(IP140B-Ac-PEG₂-Gly-Leu-Gly-m), N-(2',2',2'-trifluoroethyl)amino)ethyl-10,11-methylenedioxycamptothecin (14)

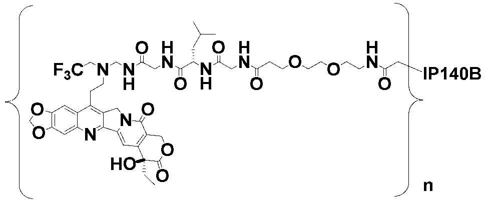

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-22** (0.84mg, 0.8mmol) was dissolved in 0.08 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 14** (3.1 mg/mL, 2 mL).

UV-HPLC calculated average value: n=7.0.

### Example 15: 7-(N-(HS627-Ac-PEG₂-Gly-Gly-Phe-Gly-ha-Ac), N-(2',2',2'-trifluoroethyl)amino)ethyl-10,11-methylenedioxycamptothecin (15)

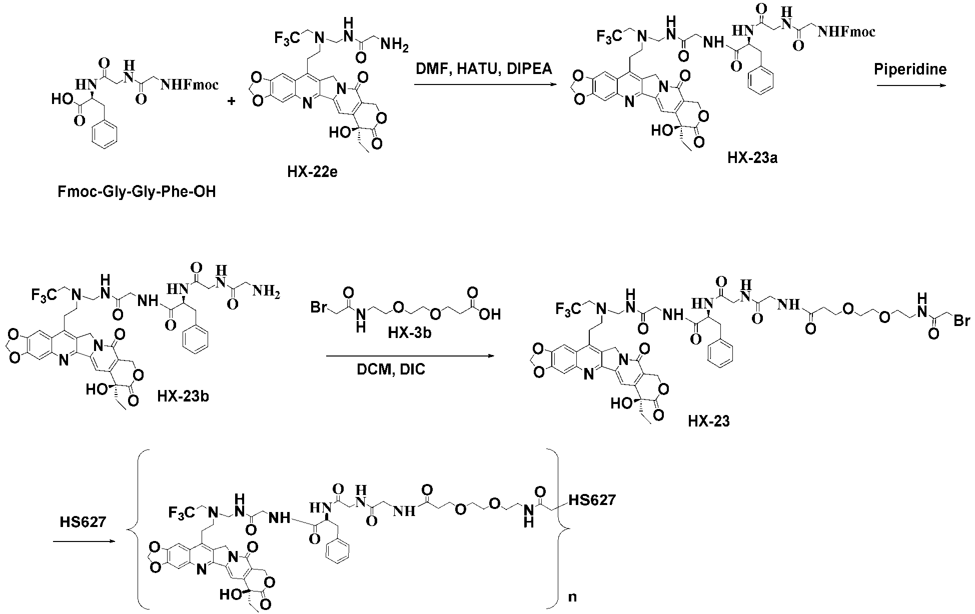

**HX-22e** (70 mg, 0.11 mmol) was taken, then 2 mL of DMF, HATU (41.8 mg, 0.11 mmol), DIPEA (14.19 mg, 0.11 mmol), and Fmoc-Gly-Gly-Phe-OH (55.11 mg, 0.11 mmol) were added. The mixture was stirred at room temperature for 6 h, then 200 mL of DCM was added for dilution. The mixture was washed with saturated sodium chloride solution, dried over anhydrous sodium sulfate, and purified by silica gel column chromatography to afford the product **HX-23** as a yellow solid (108 mg, yield 85%); LCMS: (M+1)⁺ 1087.25 (theoretical value: 1086.37).

**HX-23a** (108mg, 0.099 mmol) was dissolved in 0.2 mL of DMF, then piperidine (77mg, 0.99mmol) was added. The mixture was stirred to react at room temperature for 1 h. Then, 30 mL of methyl tert-butyl ether was added to the reaction solution. The mixture was centrifuged (5 min, 10,000 r/min), and the supernatant was removed, and the solvent was evaporated under reduced pressure to afford the product **HX-23b** as a solid (43 mg, yield 50%), which was directly used in the next step without further purification; LCMS: (M+1)⁺ 865.29 (theoretical value: 864.31).

In a 10 mL single-neck flask, DCM (2 mL), **HX-23b** (43 mg, 0.049 mmol), **HX-3b** (15 mg, 0.049 mmol) and DIC (6.27 mg, 0.049 mmol) were added in sequence. The mixture was stirred to react at room temperature for 90 min. The reaction solution was injected into a 25 g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30min). The product was lyophilized to afford **HX-23** as a yellow solid (21 mg, yield 37.5%); LCMS: (M+1)⁺ 1144.28 (theoretical value: 1143.32).

HS627 antibody (20.0 mg/mL, 10 mg, 0.066 mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-23** (0.92 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 15** (3.2 mg/mL, 2 mL).

UV-HPLC calculated average value: n=6.2.

### Example 16: 7-(N-(IP140B-Ac-PEG₂-Gly-Gly-Phe-Gly-ha-Ac), N-(2',2',2'-trifluoroethyl)amino)ethyl-10,11-methylenedioxycamptothecin (16)

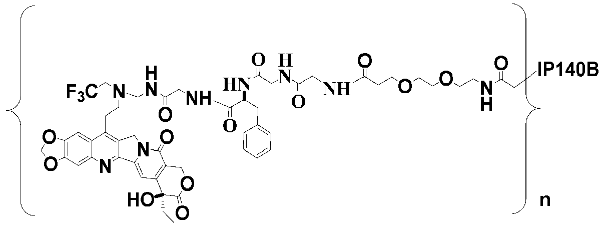

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-23** (0.92 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 16** (3.3mg/mL, 2 mL).

UV-HPLC calculated average value: n=6.5.

### Example 17: 7-(N-(HS627-Ac-PEG₂-Gly-Val-Ala-Gly-m), N-(2',2',2'-trifluoroethyl)amino)ethyl-10,11-methylenedioxycamptothecin (17)

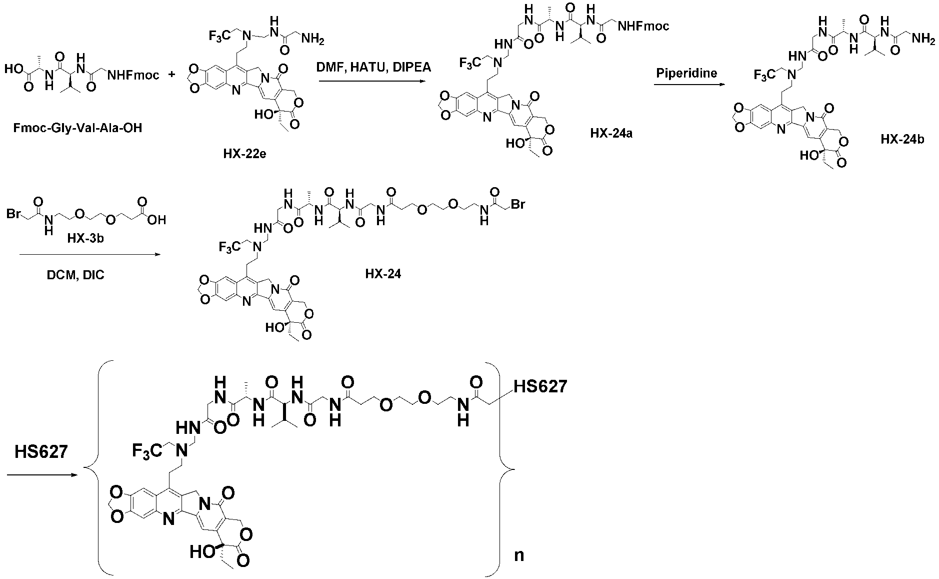

**HX-22e** (70 mg, 0.11 mmol) was taken, then 2 mL of DMF, HATU (41.8 mg, 0.11 mmol), DIPEA (14.19 mg, 0.11 mmol), and Fmoc-Gly-Val-Ala-OH (51.37 mg, 0.11 mmol) were added. The mixture was reacted at room temperature for 6 h, then 200 mL of DCM was added for dilution. The mixture was washed with saturated sodium chloride solution, dried over anhydrous sodium sulfate, and purified by silica gel column chromatography to afford the product **HX-25a** as a yellow solid (100.1 mg, yield 87%); LCMS: (M+1)⁺ 1053.06 (theoretical value: 1052.39).

**HX-25a** (100.1 mg, 0.095 mmol) was dissolved in 0.2 mL of DMF, and piperidine (74 mg, 0.95 mmol) was added. The mixture was stirred at room temperature for 1 h, then 30 mL of methyl tert-butyl ether was added. The mixture was centrifuged (5 min, 10,000 r/min), and the supernatant was removed, and the solvent was evaporated under reduced pressure to afford the product **HX-24b** as a solid (49 mg, yield 62%), which was directly used in the next step; LCMS: (M+1)⁺ 831.27 (theoretical value: 830.32).

In a 10 mL single-neck flask, DCM (2 mL), **HX-24b** (49 mg, 0.059 mmol), **HX-3b** (17.6 mg, 0.059 mmol) and DIC (7.43 mg, 0.059 mmol) were added in sequenc. The mixture was stirred to react at room temperature for 90 min. The reaction solution was injected into a 25 g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30min). The product was lyophilized to afford **HX-24** as a yellow solid (15 mg, yield 22%); LCMS: (M+1)⁺ 1110.28 (theoretical value: 1109.33).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-24** (0.89mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 17**(3.1mg/mL, 2 mL).

UV-HPLC calculated average value: n=6.3.

### Example 18: 7-(N-(IP140B-Ac-PEG₂-Gly-Val-Ala-Gly-m), N-(2',2',2'-trifluoroethyl)amino)ethyl-10,11-methylenedioxycamptothecin (18)

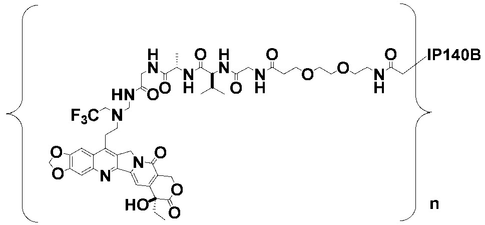

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-24** (0.89mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 18** (3.3mg/mL, 2 mL).

UV-HPLC calculated average value: n=6.6.

### Example 19: 7-(N-(HS627-Ac-PEG₂-Gly-Val-Ala-Gly-ha-Ac), N-(2',2',2'-trifluoroethyl)amino)ethyl-10,11-methylenedioxycamptothecin (19)

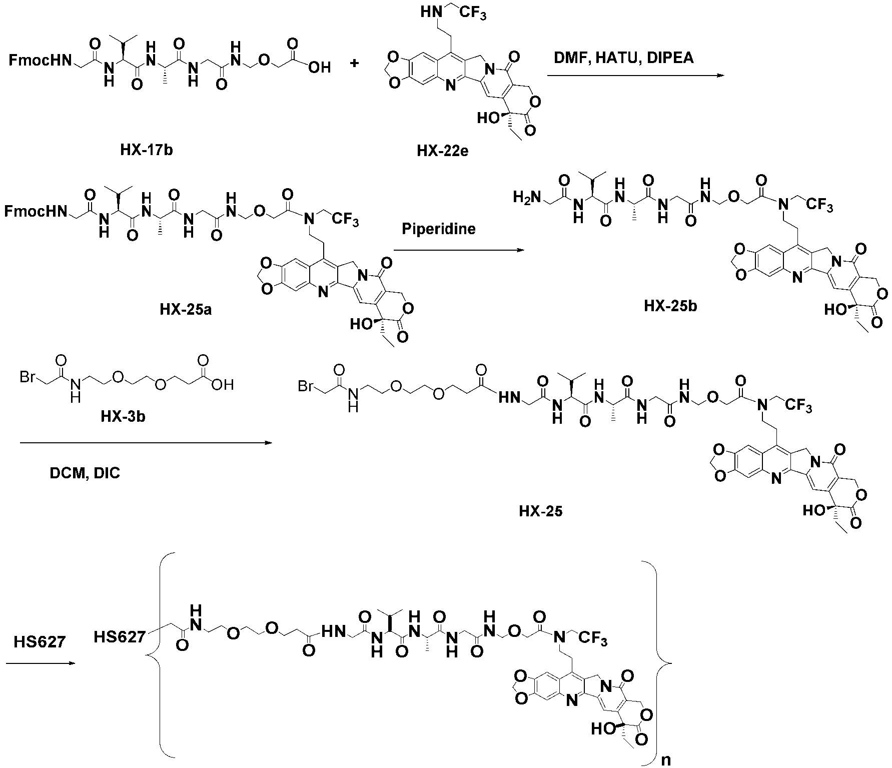

**HX-22e** (50 mg, 0.096 mmol) was taken, then 2 mL of DMF, HATU (36.7 mg, 0.096 mmol), DIPEA (25 mg. 0.193 mmol) and **HX-17b** (118 mg, 0.096 mmol) were added, and the mixture was reacted at room temperature for 15 h, then 200 mL of DCM was added for dilution. The mixture was washed with saturated sodium chloride solution, dried over anhydrous sodium sulfate, and purified by silica gel column chromatography to afford the product **HX-25a** as a yellow solid (92 mg, yield 85%); LCMS: (M+1)⁺ 1111.10 (theoretical value: 1110.39).

**HX-25a** (92 mg, 0.082 mmol) was dissolved in 0.2 mL of DMF, then piperidine (64 mg, 0.82 mmol) was added. The mixture was stirred at room temperature for 1 h, then 30 mL of methyl tert-butyl ether was added. The mixture was centrifuged (5 min, 10,000 r/min), and the supernatant was removed, and the solvent was evaporated under reduced pressure to afford the product **HX-25b** as a solid (52 mg, yield 71%), which was directly used in the next step; LCMS: (M+1)⁺ 889.72 (theoretical value: 888.86).

In a 10 mL single-neck flask, DCM (2 mL), **HX-25b** (52 mg, 0.058 mmol), **HX-3b** (17.74 mg, 0.058 mmol) and DIC (7.4 mg, 0.058 mmol) were added in sequence. The mixture was stirred to react at room temperature for 90 min. The reaction solution was injected into a 25 g C18 pre-column ((pre-equilibrated with acetonitrile, then with water, the water phase contains 0.1% TFA), then eluted via medium-pressure reverse-phase C18 chromatography (gradient: 5% to 40% acetonitrile in water over 30min). The product was lyophilized to afford **HX-25** as a yellow solid (15 mg, yield 22%); LCMS: (M+1)⁺ 1168.27 (theoretical value: 1167.34).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-25** (0.93 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 19**(3.0mg/mL, 2 mL).

UV-HPLC calculated average value: n=7.3.

### Example 20: 7-(N-(IP140B-Ac-PEG₂-Gly-Val-Ala-Gly-ha-Ac), N-(2',2',2'-trifluoroethyl)amino)ethyl-10,11-methylenedioxycamptothecin (20)

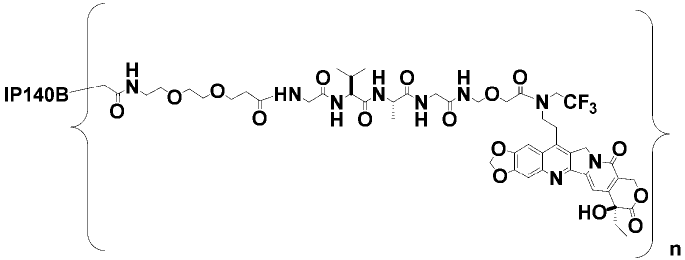

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-25**(0.93 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 20** (3.3mg/mL, 2 mL).

UV-HPLC calculated average value: n=7.5.

### Example 21: 20-O-(HS627-Ac-PEG₂-Gly-Val-Ala-Gly-NHNHC)-7-(N-(2',2',2'-trifluoroethyl)amino)ethyl-10,11-methylenedioxycamptothecin (21)

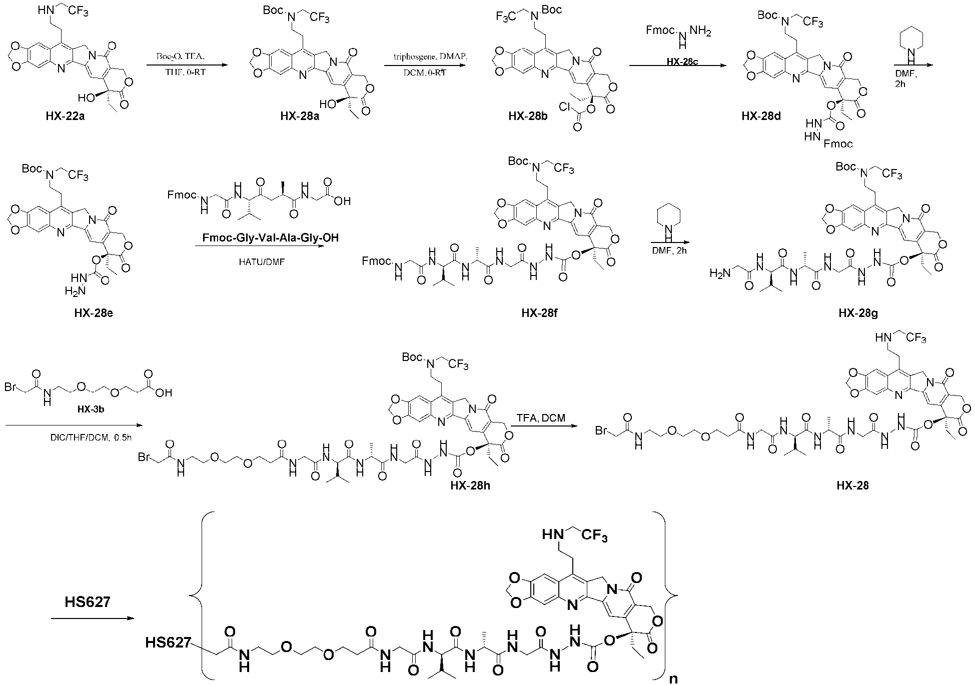

In a 50 mL single-neck flask, **HX-22e** (200 mg, 0.386 mmol) and 3 mL of dichloromethane were added, then Boc anhydride (92.8 mg, 0.425 mmol) and TEA (86.1 mg, 0.851 mmol) were added in sequence. The mixture was stirred at room temperature for 6 h, the solvent was evaporated under reduced pressure, and the residue was purified by silica gel column chromatography to afford the product **HX-28a** as a pale yellow solid (213.2 mg, yield 89.3%); LCMS: (M+1)⁺ 618.20 (theoretical value: 617.58).

In a 50 mL single-neck flask, **HX-28a** (100 mg, 0.162 mmol), DMAP (98.9 mg, 0.81 mmol) and 2 mL of dichloromethane were added in sequence. The mixture was stirred to react at 0 °C for 5 minutes, then 2 mL of dichloromethane and triphosgene (38.6 mg, 0.131 mmol) were add. The mixture was reacted at room temperature for 2 h to afford a yellow clear solution of **HX-28b.** Then, **HX-26c** (82.3 mg, 0.324 mmol) was added. The mixture was stirred to react at room temperature for 3 h, and purified by silica gel column chromatography to afford the product **HX-28d** as a pale yellow solid (123.5 mg, yield 85.3%); LCMS: (M+1)⁺ 898.29 (theoretical value: 897.27).

In a 10 mL single-neck flask, **HX-28d** (100 mg, 0.11 mmol) was added and dissolved with 1 mL of DMF. Piperidine (94.8 mg, 1.1 mmol) was then added, and the mixture was reacted at room temperature for 0.5 h. The reaction solution was added dropwise to 20 mL of methyl tert-butyl ether. The mixture was centrifuged, and the supernatant was removed, and the residue was dried to afford **HX-28e** (65.5 mg, yield 87%); LCMS: (M+1)⁺ 676.22 (theoretical value: 675.22).

**HX-28e** (50 mg, 0.074 mmol) was taken and then 2 mL of DMF, HATU (33.8 mg, 0.089 mmol), DIPEA (11.5 mg, 0.089 mmol), and Fmoc-Gly-Val-Ala-Gly-OH (38.8 mg, 0.074 mmol) were added. The mixture was reacted at room temperature for 2 h, then 200 mL of DCM was added for dilution. The mixture was washed with saturated sodium chloride solution, dried over anhydrous sodium sulfate, and purified by silica gel column chromatography to afford the **HX-28f** as a yellow solid (51.3 mg, yield 58.7%); LCMS: (M+1)⁺ 1181.19 (theoretical value: 1180.44).

In a 10 mL single-neck flask, **HX-28f** (40 mg, 0.034 mmol) was added and dissolved with 1 mL of DMF. Piperidine (28.7 mg, 0.34 mmol) was then added, and the mixture was reacted at room temperature for 0.5 h, then 20 mL of methyl tert-butyl ether was added. The mixture was centrifuged, and the supernatant was removed, and the residue was dried to afford **HX-28g** (25.8 mg, yield 79.5%); LCMS: (M+1)⁺ 958.37 (theoretical value: 959.37).

In a 10 mL single-neck flask, DCM (1 mL), **HX-28g** (20.2 mg, 0.021 mmol), **HX-3b** (7.6 mg, 0.026 mmol), and DIC (3.2 mg, 0.026 mmol) were added in sequence. The mixture was stirred at room temperature for 30 min to afford a clear yellow solution of **HX-28h.** 100 µL of trifluoroacetic acid was added, and the mixture was stirred to react at room temperature for 30 min. The reaction solution was injected into a 25-g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30 min). The product was lyophilized to afford the **HX-28** as a yellow solid (4.1 mg, yield 17%); LCMS: (M+1)⁺ 1138.95 (theoretical value: 1137.33).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-28** (0.91 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 21** (3.2 mg/mL, 2 mL).

UV-HPLC calculated average value: n=7.1.

### Example 22: 20-O-(IP140B-Ac-PEG₂-Gly-Val-Ala-Gly-NHNHC)-7-(N-(2',2',2'-trifluoroethyl)amino)ethyl-10,11-methylenedioxycamptothecin (22)

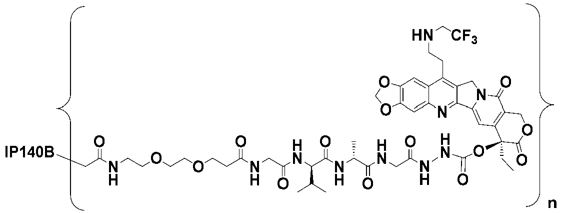

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-28** (0.91 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 22**(3.1 mg/mL, 2 mL).

UV-HPLC calculated average value: n=7.0.

### Example 23: 20-O-(HS627-Ac-PEG₄-daP-NHNHC)-7-(N-(2',2',2'-trifluoroethyl)amino)ethyl-10,11-methylenedioxycamptothecin (23)

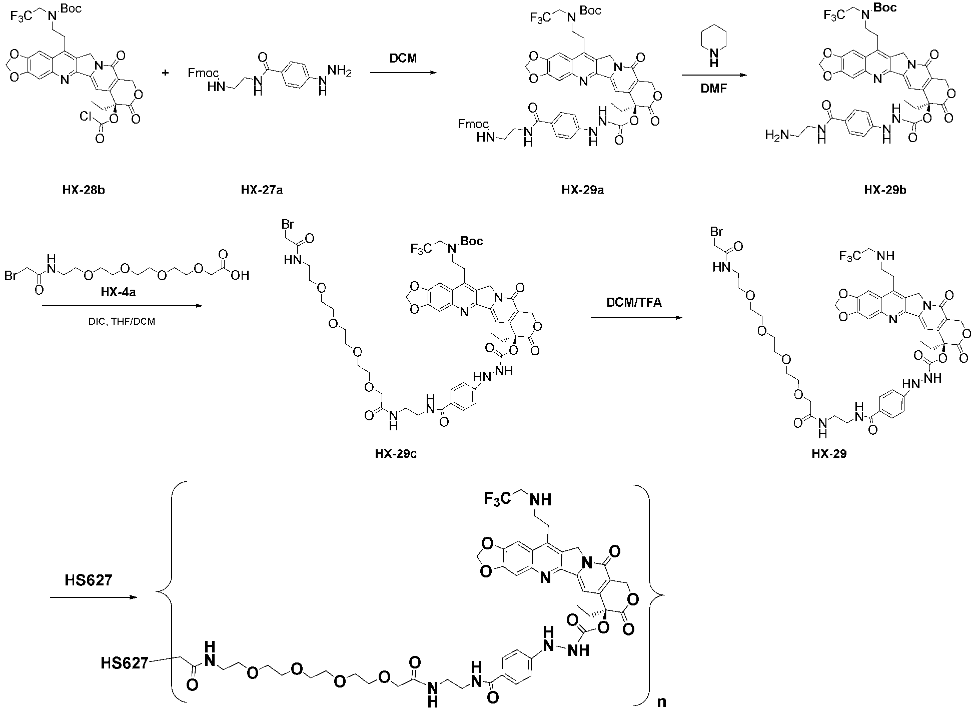

In the yellow clear solution of **HX-28b, HX-27a** (113.8 mg, 0.274 mmol) was directly added. The mixture was stirred at room temperature for 3 h, and purified by silica gel column chromatography to afford the **HX-29a** as a pale yellow solid (125.6 mg, yield 86.7%); LCMS: (M+1)⁺ 1060.05 (theoretical value: 1059.36).

In a 10 mL single-neck flask, **HX-27b** (125.6 mg, 0.118 mmol) was added and dissolved with 1 mL of DMF. Piperidine (100.9 mg, 1.18 mmol) was then added, and the mixture was reacted at room temperature for 0.5 h, then 20 mL of methyl tert-butyl ether was added. The mixture was centrifuged, and the supernatant was removed, and the residue was dried to afford **HX-29b** (93.5 mg, 94.2%); LCMS: (M+1)⁺ 838.30 (theoretical value: 837.29).

In a 10 mL single-neck flask, DCM (2 mL), **HX-29b** (70 mg, 0.084 mmol), **HX-4a** (37.3 mg, 0.1 mmol), and DIC (12.6 mg, 0.1 mmol) were added in sequence. The mixture was stirred at room temperature for 30 min to afford a clear yellow solution of **HX-29c.** Then, 200 µL of trifluoroacetic acid was added, and the mixture was stirred to react at room temperature for 30 min. The reaction solution was injected into a 25-g C18 pre-column (pre-equilibrated with acetonitrile, then with water, the water phase contains 0.1% TFA), followed by elution via medium-pressure reverse-phase C18 chromatography (gradient: 5% to 40% acetonitrile in water over 30 min). The product was lyophilized to afford the **HX-29** as a yellow solid (20.2 mg, yield 22.1%); LCMS: (M+1)⁺ 1092.29 (theoretical value: 1091.89).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-29**(0.87mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 23** (3.3 mg/mL, 2 mL).

UV-HPLC calculated average value: n=7.3.

### Example 24: 20-O-(IP140B-Ac-PEG₄-daP-NHNHC)-7-(N-(2',2',2'-trifluoroethyl)amino)ethyl-10,11-methylenedioxycamptothecin (24)

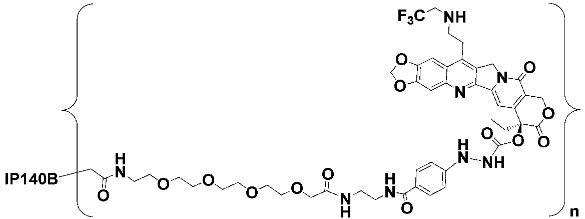

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-29** (0.87 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 24** (3.2 mg/mL, 2 mL).

UV-HPLC calculated average value: n=7.5.

### Example 25: 7-(N-(HS627-Qx-PEG₂-Gly-Lys-PABC), N-(2',2'-difluoroethyl)amino)ethyl-10,11-methylenedioxycamptothecin (25)

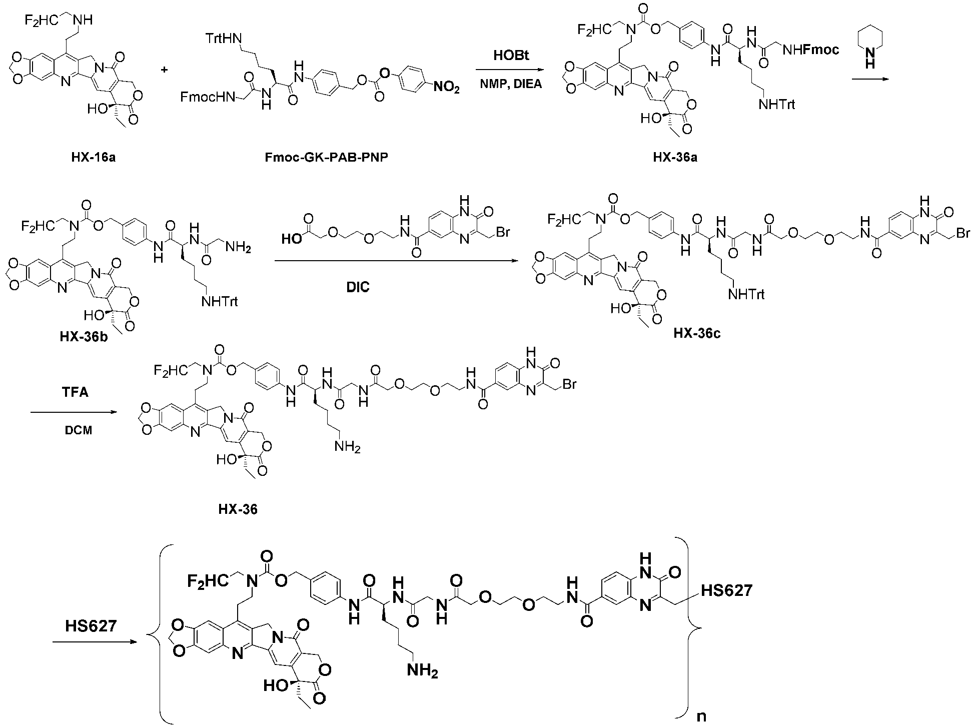

N-(2',2'-difluoroethyl)aminoethyl-10,11-methylenedioxycamptothecin (0.05 g, 0.1 mmol), 1 mL of NMP, DIPEA (25.9 mg, 0.2 mmol), Fmoc-GK-PAB-PNP (93.9 mg, 0.1 mmol), HOBt (20.3 mg, 0.15 mmol) were taken. The mixture was stirred to react at room temperature for 4 h to afford **HX-36a.** The reaction solution was directly used in the next step; LCMS: (M+1)⁺ 1298.41 (theoretical value: 1297.50).

In a 10 mL reaction vial, **HX-36a** (130 mg, 0.1 mmol) and piperidine (85.3 mg, 1 mmol) were added in sequence. The mixture was stirred to react at room temperature for 1 h, then 30 mL of methyl tert-butyl ether was added. The mixture was centrifuged, and the supernatant was removed, and the solvent was evaporated under reduced pressure to afford the product **HX-36b** as a solid (78.2 mg, yield 72.6%), which was directly used in the next step; LCMS: (M+1)⁺ 1076.39 (theoretical value: 1075.43).

In a 10 mL single-neck flask, DCM (2 mL), **HX-36b** (78.2 mg, 0.073 mmol), 2-bromomethyl-3-oxo-dihydroquinoxaline-6-formyl-3,6-dioxa-octanoic acid (38.6 mg, 0.087 mmol), and DIC (11 mg, 0.087 mmol) were added in sequence. The mixture was stirred at room temperature for 90 min, followed by the addition of 0.1 mL of TFA and continued to react for 30 min. The reaction solution was injected into a 25-g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30 min). The product was lyophilized to afford **HX-36** as a yellow solid (46.3 mg, yield 50.5%); LCMS: (M+1)⁺ 1258.10 (theoretical value: 1256.33).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-36** (1 mg, 0.8 mmol) was dissolved in 0.1 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 25** (3.2 mg/mL, 2 mL).

UV-HPLC calculated average value: n=6.3.

### Example 26: 7-(N-(IP140B-Qx-PEG₂-Gly-Lys-PABC), N-(2',2'-difluoroethyl)amino)ethyl-10,11-methylenedioxycamptothecin (26)

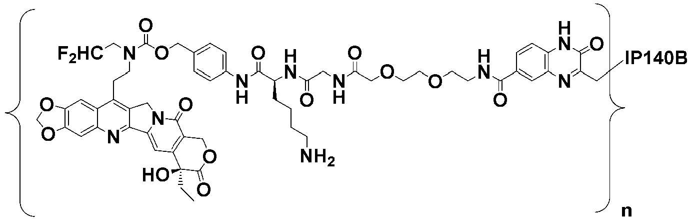

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-36** (1 mg, 0.8 mmol) was dissolved in 0.1 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 26** (3.2 mg/mL, 2 mL).

UV-HPLC calculated average value: n=6.6.

### Assay 1: In vitro inhibitory activity of ADC against tumor cell growth

Method for *in vitro* inhibitory activity assay of ADC: Human esophageal cancer cells OE-33, lung cancer cells NCI-H1975, and breast cancer cells MDA-MB-231 used for activity assays were respectively cultured in RPMI1640 (Cellmax), RPMI1640 (Cellmax), and DMEM (Cellmax) media containing 10% fetal bovine serum (Cellmax) to the exponential growth phase. After trypsinization, the cells were centrifuged, and the supernatant was discarded. The cells were resuspended with media and diluted to 3x10⁴ cells/mL, 0.5x10⁴ cells/mL and 1.5x10⁴ cells/mL respectively. Then, 100 µL of each cell suspension was added to a 96-well cell culture plate, and the plate was returned to an incubator at 37°C with 5% CO₂ for overnight culture. The next day, the ADC to be tested was diluted to 2000 nM, 400 nM, 80 nM, 16 nM, 3.2 nM, 0.64 nM, 0.128 nM, and 0.026 nM using culture medium. The diluted ADC was added to a 96-well cell culture plate at 100 µL per well, with 3 replicates set for each concentration. For the negative control without ADC and the blank control, 100 µL of culture medium was added to each well. After the sample addition was finished, the plate was returned to the incubator at 37°C with 5% CO₂ for further incubation for 6 days. Upon completion of incubation, the cell culture plate was removed, and the medium in the plate was aspirated using a pipette. 100 µL of medium containing 10% CCK-8 was added to each well, followed by incubation at 37°C for 3 h. After incubation, the plate was removed, protected from light, placed in a microplate reader, and the absorbance was measured at a test wavelength of 450 nm with a reference wavelength of 630 nm. Based on the absorbance values, the IC₅₀ (Table 1) was calculated using four-parameter regression in GraphPad. The corresponding ADC drug containing Dxd was used as a positive control drug, which has the following structure: 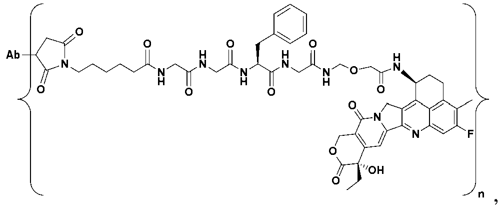 wherein, Ab is the IP140B antibody.

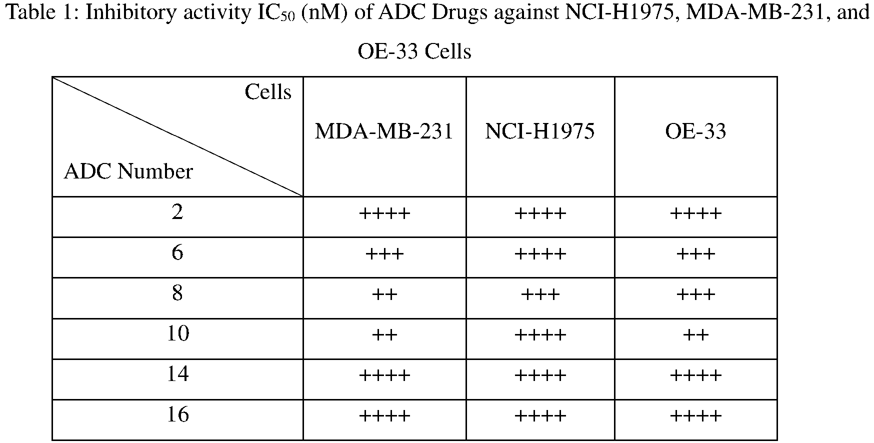

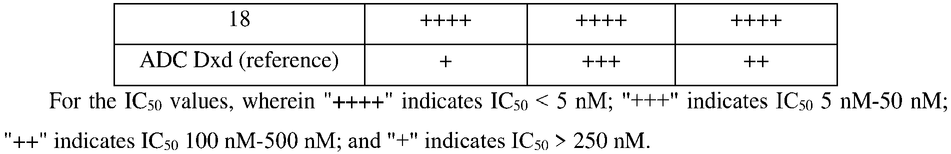

The ADC compounds of the examples in the present disclosure all have good inhibitory activities against NCI-H1975, MDA-MB-231, and OE-33 cells. Their inhibitory activity IC₅₀ against these cells are all lower than 250 nM. Some ADC compounds such as ADC2, 14, 16 and 18 even all have inhibitory activities IC₅₀against these cancer cells of lower than 50 nM.

### Assay 2: In vivo inhibitory activity of ADC against tumor growth

Method for *in vivo* inhibitory activity assay of ADC: Human lung cancer cell NCI-H292, Human non-small cell lung cancer cell Calu-6, Human lung cancer cell NCI-H1975, Human esophageal squamous cell carcinoma cell KYSE-150, Human ovarian cancer cell ES-2 and Human breast cancer cell MDA-MB-231 were cultured *in vitro* as a monolayer. When the cell saturation was 80%-90%, they were digested with trypsin-EDTA, centrifuged, and the supernatant was discarded. The cells were resuspended with PBS, and the cell suspension was adjusted to a suitable concentration. NCI-H292, Calu-6, NCI-H1975, KYSE-150 and MDA-MB-231cells (2⁻¹⁰x10⁶ cells/0.1 mL) were subcutaneously inoculated into BALB/c nude mice. ES-2 cells were subcutaneously inoculated into NOD-SCID mice. The animals and the growth of transplanted tumors were regularly observed. When the tumor volume was grown to about 100-200 mm³, random grouping was performed according to tumor volume and body weight, namely the vehicle control group (normal saline) and the ADC administration group (dissolved in normal saline), with 6 animals per group. Administration was performed by intravenous injection at a dosing frequency of Q4D for a total of 2 times (the first administration was recorded as Day 1, and the second administration was on Day 5). Experimental grouping and dosing settings were shown in Table 2. Tumor major diameter a (mm), minor diameter b (mm), and mice body weight were measured with a vernier caliper twice a week. Tumor volume (V) was calculated according to the following formula: V = 1/2xaxb2 (mm³), wherein a and b represents the tumor length and width, respectively. Growth curves were plotted, and finally, tumors were excised and weighed. Statistical analysis was performed using GraphPad Prism software based on tumor volume and body weight data of tumor-bearing mice at the end of the trial to afford tumor inhibition results. In the figures, "\" in the control group indicates no results, with tumor status unmeasured; the experimental group had no corresponding tumors. "Tumor volume reduced to 0" and "tumor disappeared to 0" indicates no tumor residue was found in the corresponding dissected animals.

**Table 2: Mouse Grouping and Administration Settings**

| Cell Model | Group/Drug Injected | Administration Dose | Tumor Growth Curves | Photographs of Tumors after Dissection |
|---|---|---|---|---|
| Calu-6 | Vehicle control group | - | Figure 1 | Figure 2 |
| | ADC Dxd | 3mg/kg weight | | |
| | | 10mg/kg weight | | |
| | ADC 12 | 3mg/kg weight | | |
| | | 10mg/kg weight | | |
| KYSE-150 | Vehicle control group | - | Figure 3 | Figure 4 |
| | ADC Dxd | 3mg/kg weight | | |
| | | 10mg/kg weight | | |
| | ADC 12 | 3mg/kg weight | | |
| | | 10mg/kg weight | | |
| ES-2 | Vehicle control group | - | Figure 5 | Figure 6 |
| | ADC Dxd | 3mg/kg weight | | |
| | | 10mg/kg weight | | |
| | ADC 12 | 3mg/kg weight | | |
| | | 10mg/kg weight | | |
| NCI-H1975 | Vehicle control group | - | Figure 7 | Figure 8 |
| | ADC Dxd | 3mg/kg weight | | |
| | | 10mg/kg weight | | |
| | ADC 16 | 3mg/kg weight | | |
| | | 6mg/kg weight | | |
| | ADC 10 | 10mg/kg weight | | |
| | ADC 8 | 3mg/kg weight | | |
| | | 10mg/kg weight | | |
| MDA-MB-231 | Vehicle control group | - | Figure 9 | Figure 10 |
| | ADC Dxd | 2mg/kg weight | | |
| | | 5mg/kg weight | | |
| | ADC 14 | 2mg/kg weight | | |
| | | 5mg/kg weight | | |
| | ADC 2 | 2mg/kg weight | | |
| | | 5mg/kg weight | | |

The above descriptions of specific exemplary embodiments of the present disclosure are for purposes of illustration and exemplification. These descriptions are not intended to limit the present disclosure to the precise forms disclosed, and it is apparent that many changes and modifications can be made in light of the above teachings. The purpose of selecting and describing exemplary embodiments is to explain the specific principles of the present disclosure and practical applications thereof, thereby enabling those skilled in the art to implement and utilize various exemplary embodiments of the present disclosure, along with various selections and modifications. The scope of the present disclosure is intended to be defined by the claims and equivalents thereof.

## Claims

1. An antibody-drug conjugate represented by Formula (I), 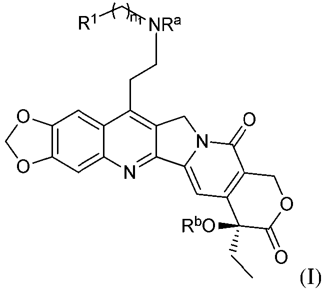 in the Formula,
R¹ is selected from halogen-substituted methyl;
R^{a} is selected from hydrogen and -L¹-NH-L^{p}-Z-Ab,
R^{b} is selected from hydrogen, -L²-NH-L^{p}-Z-Ab and -L³-NH-Z-Ab, provided that R^{a}, R^{b} are not both hydrogen;
wherein,
L¹ is selected from -C(=O)-L¹¹-L¹²-L¹³- and -CH₂-NR²-L¹⁴-CHR³-;
L¹¹, L¹², L¹³ are each independently selected from -O-, C₁-C₃ alkylene and phenyl;
R² is selected from hydrogen, deuterium, C₁-C₆ alkyl, C₁-C₆ alkoxyl, acyl, sulfonyl;
L¹⁴ is selected from -C(=O)- and C₁-C₃ alkylene;
R³ is selected from hydrogen, deuterium, C₁-C₆ alkyl, C₁-C₆ alkoxyl and phenyl-substituted C₁-C₆ alkyl;
L² is selected from-C(=O)-NH-L²¹-CHR⁴-;
_{L}²¹ is selected from -C(=O)- and -NH-C(=O)-;
R⁴ is selected from hydrogen, deuterium, C₁-C₆ alkyl and phenyl-substituted C₁-C₆ alkyl;
L³ is selected from -C(=O)-NR⁵-NR⁶-L³¹-L³²-;
R⁵, R⁶ are each independently selected from hydrogen, deuterium and C₁-C₆ alkyl;
L³¹ is selected from -C(=O), -(C₁-C₃ alkylene)-C(=O), -5-8 membered aryl-C(=O)-, -5-8 membered azaheteroaryl-C(=O)- and -O-(C₁-C₃ alkylene)-C(=O)-;
L³² is selected from -NH-(C₁-C₃ alkylene), -N(CH₃)₂-(C₁-C₃ alkylene) and -NH-(C₁-C₃ alkylene)-NH-;
L^{P} is a peptide residue consisting of 2 to 7 amino acids;
Z is selected from -L^{z}-L^{j}-, wherein, L^{z} is selected from -C(=O)-C₁-C₈ alkylene, -C(=O)-CH₂O-(CH₂CH₂O)₂₋₅-CH₂CH₂NH- or -C(=O)-(CH₂CH₂O)₂₋₆-CH₂CH₂NH-, L^{j} is a linker that can conjugate to an antibody;
n₁ is selected from an integer of 1-3;
Ab is an antibody.

2. The antibody-drug conjugate according to claim 1, wherein, R¹ is selected from methyl substituted by fluorine, chlorine or bromine;
preferably, R¹ is selected from fluorine-substituted methyl;
preferably, R¹ is selected from fluoromethyl, difluoromethyl and trifluoromethyl;
preferably, R¹ is selected from difluoromethyl and trifluoromethyl;
preferably, n₁ is 1.

3. The antibody-drug conjugate according to claim 1 or 2, wherein, L¹¹, L¹², L¹³ are each independently selected from -O-, methylene and phenyl;
preferably, L¹¹-L¹²-L¹³ is selected from -O-CH₂-phenyl- and -CH₂-O-CH₂-;
preferably, R² is selected from hydrogen, deuterium, C₁-C₆ alkyl, C₁-C₆ alkoxyl, -C(=O)C₁-C₆ alkyl and -S(=O)₂C₁-C₆ alkyl;
preferably, R² is selected from hydrogen, deuterium, C₁-C₃ alkyl, C₁-C₃ alkoxyl, -C(=O)C₁-C₃ alkyl and -S(=O)₂C₁-C₃ alkyl;
preferably, R² is selected from hydrogen, deuterium, methyl, ethyl, methoxy, ethoxy, formyl, acetyl, methanesulfonyl and ethanesulfonyl;
preferably, R² is selected from hydrogen, methyl, methoxy, formyl and methanesulfonyl;
preferably, R² is hydrogen;
preferably, L¹⁴ is selected from -C(=O)- and C₁-C₃ alkylene;
preferably, L¹⁴ is selected from -C(=O)- and methylene;
preferably, R³ is selected from hydrogen, deuterium, C₁-C₆ alkyl, C₁-C₆ alkoxyl and phenyl-substituted C₁-C₆ alkyl;
preferably, R³ is selected from hydrogen, C₁-C₆ alkyl and phenyl-substituted C₁-C₆ alkyl;
preferably, R³ is selected from hydrogen, C₁-C₄ alkyl and phenyl-substituted C₁-C₃ alkyl;
preferably, R³ is selected from hydrogen, methyl, ethyl, propyl, isopropyl, butyl, isobutyl, phenylmethyl and phenylethyl;
preferably, R³ is selected from hydrogen, methyl, isopropyl, isobutyl and phenylmethyl;
preferably, R³ is hydrogen;
preferably, L¹ is selected from -C(=O)-O-CH₂-phenyl-, -C(=O)-CH₂-O-CH₂-, -CH₂-NH-C(=O)-CH₂- and -CH₂-NH-CH₂-CH₂-;
preferably, L¹ is selected from -C(=O)-O-CH₂-phenyl-, -C(=O)-CH₂-O-CH₂- and -CH₂-NH-C(=O)-CH₂-;
preferably, L¹ is selected from -C(=O)-O-CH₂-phenyl- and -C(=O)-CH₂-O-CH₂-.

4. The antibody-drug conjugate according to any one of claims 1-3, wherein, R⁴ is selected from hydrogen, deuterium, C₁-C₆ alkyl and phenyl-substituted C₁-C₆ alkyl;
preferably, R⁴ is selected from hydrogen, deuterium, C₁-C₃ alkyl and phenyl-substituted C₁-C₃ alkyl;
preferably, R⁴ is selected from hydrogen, deuterium, methyl, isopropyl, isobutyl and phenylmethyl;
preferably, R⁴ is selected from hydrogen;
preferably, L² is selected from -C(=O)-NH-C(=O)-CH₂- and -CH₂-NH-NH-C(=O)-CH₂-;
preferably, L² is -CH₂-NH-NH-C(=O)-CH₂-;
preferably, R³, R⁶ are each independently selected from hydrogen, deuterium and C₁-C₃ alkyl;
preferably, R⁵, R⁶ are each independently selected from hydrogen, deuterium and methyl;
preferably, R⁵ is hydrogen, R⁶ is hydrogen;
preferably, L³¹ is selected from -C(=O), -methylene-C(=O), -phenyl-C(=O)-, -pyridyl-C(=O)- and -O-CH₂-C(=O)-;
preferably, L³² is selected from -NH-CH2-CH2-, -N(CH₃)₂-CH₂-CH₂- and -NH-CH₂-NH-;
preferably, L³ is -C(=O)-NH-NH-phenyl-C(=O)-NH-CH₂-CH₂-.

5. The antibody-drug conjugate according to any one of claims 1-4, wherein, the amino acid is selected from phenylalanine (Phe), glycine (Gly), valine (Val), alanine (Ala), leucine (Leu), lysine (Lys), citrulline (Cit), and asparagine (Asn);
preferably, L^{p} is selected from peptide residues consisting of 2-5 amino acids selected from phenylalanine (Phe), glycine (Gly), valine (Val), alanine (Ala), leucine (Leu), lysine (Lys), citrulline (Cit), and asparagine (Asn);
preferably, L^{p} is selected from peptide residues consisting of 2-5 amino acids selected from phenylalanine (Phe), glycine (Gly), valine (Val), alanine (Ala), leucine (Leu), lysine (Lys);
preferably, L^{p} is selected from -Val-Cit-, -Val-Ala-, -Gly-Val-Ala-, -Gly-Val-Ala-Gly-, -Gly-Lys-, -Gly-Gly-Lys-, -Gly-Gly-Lys-Gly-, -Val-Ala-, -Ala-Ala-Asn-, -Gly-Leu-, -Gly-Gly-Leu-, -Gly-Gly-Leu-Gly-, -Gly-Phe-, -Gly-Gly-Phe- and -Gly-Gly-Phe-Gly-;
preferably, L^{p} is selected from 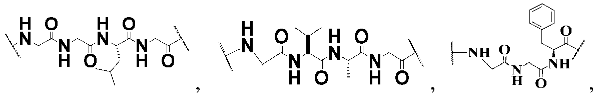 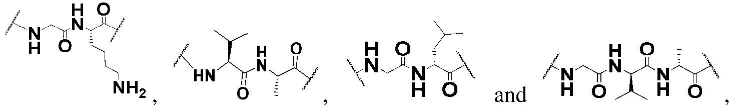 the carbonyl terminus thereof is linked to -NH- and the other terminus is linked to Z;
preferably, L^{j} is selected from 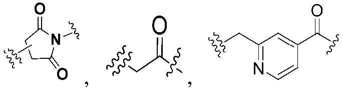 and 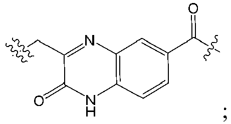 the position represented by " 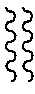 " is linked to the antibody, and the position represented by " 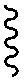 " is linked to the L^{z} group;
preferably, L^{z} is selected from -C(=O)-C₁-C₈ alkylene, -C(=O)-CH₂O-(CH₂CH₂O)₂₋₅-CH₂CH₂NH- and -C(=O)-(CH₂CH₂O)₂₋₆-CH₂CH₂NH-;
preferably, L^{z} is selected from -C(=O)-C₁-C₈ alkylene, -C(=O)-CH₂O-(CH₂CH₂O)₂₋₃-CH₂CH₂NH- and -C(=O)-(CH₂CH₂O)₂₋₄-CH₂CH₂NH-;
preferably, L² is selected from -C(=O)-(CH₂CH₂O)₂-CH₂CH₂NH-, -C(=O)-CH₂O-CH₂CH₂O-CH₂CH₂NH- and -C(=O)-CH₂O-(CH₂CH₂O)₃-CH₂CH₂NH;
preferably, Z is selected from 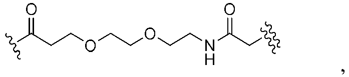 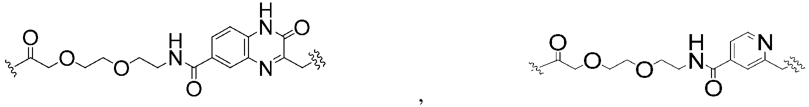
and 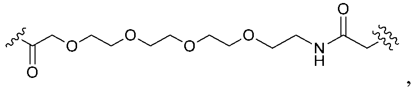 the position represented by " 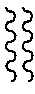 " is linked to the antibody, and the position represented by " 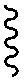 " is linked to the L^{p} group or L³ group.

6. The antibody-drug conjugate according to any one of claims 1-5, wherein, the antibody is an antibody against tumor-associated antigen;
preferably, the antibody against tumor-associated antigen is selected from anti-Her2 antibody, anti-Trop2 antibody, anti-B7H3 antibody, anti-5T4 antibody, anti-Nectin-4 antibody, anti-CD20 antibody, and anti-ROR1 antibody.

7. The antibody-drug conjugate according to any one of claims 1-6, the antibody-drug conjugate represented by Formula (I) has a structure represented by Formula (1-1), Formula (I-2), Formula (I-3), Formula (I-4), Formula (I-5) or Formula (I-6), 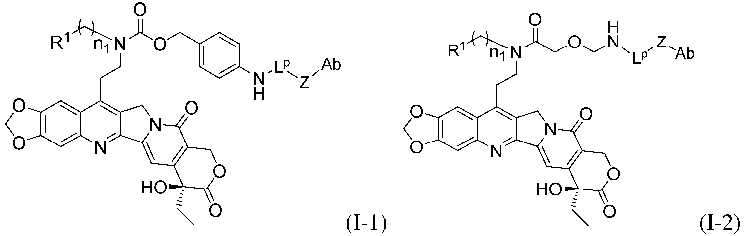 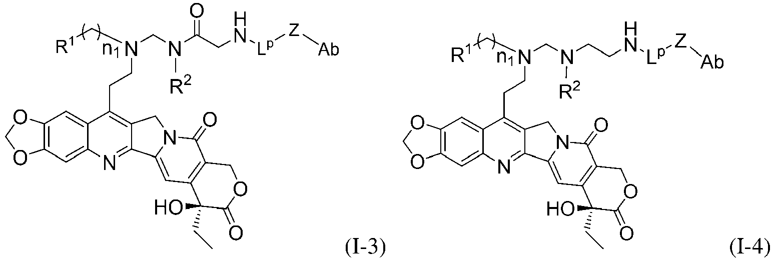
in Formula (I-1), Formula (I-2), Formula (I-3), Formula (I-4), R¹, R², n₁, L^{p}, Z, Ab are defined the same as the compound of Formula (I) at each occurrence; 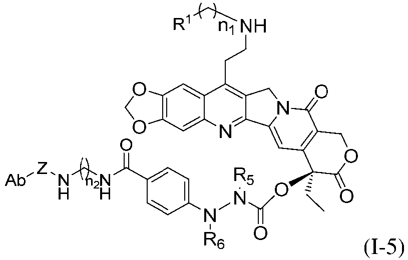
in Formula (I-5), R¹, R⁵, R⁶, n₁, Ab are each defined the same as the compound of Formula (I);
n₂ is selected from 1 or 2;
Z is selected from -L^{z}-L^{j}-, wherein, L^{j} is selected from 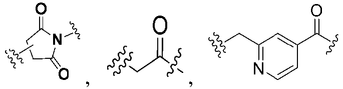
and 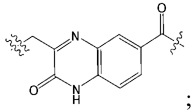 the position represented by 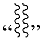 is linked to the antibody, and the position represented by 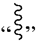 is linked to the L^{Z} group;
preferably, L^{z} is selected from -C(=O)-CH₂O-(CH₂CH₂O)₁₋₅-CH₂CH₂NH-;
preferably, L^{z} is selected from -C(=O)-CH₂O-(CH₂CH₂O)₃₋₅-CH₂CH₂NH-;
preferably, L^{z} is selected from -C(=O)-CH₂O-(CH₂CH₂O)₃-CH₂CH₂NH-;
preferably, Z is selected from 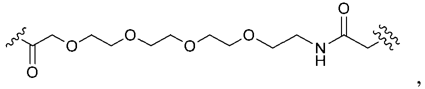 the position represented by 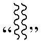 is linked to the antibody, and the position represented by 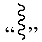 is linked to the L^{p} group or L³ group; 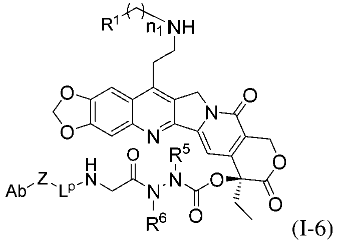
in Formula (I-6), R¹, R⁵, R⁶, n₁, L^{p}, Z, Ab are each defined the same as the compound of Formula (I).

8. The following antibody-drug conjugate: 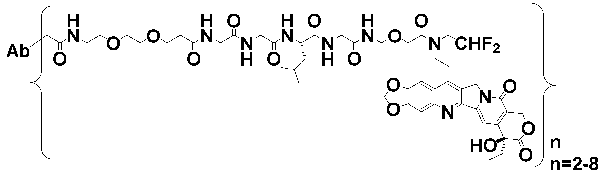 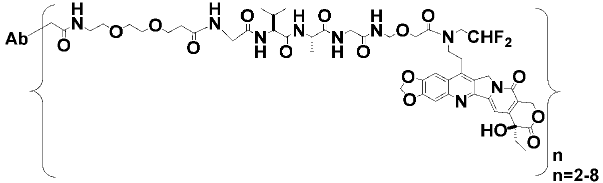 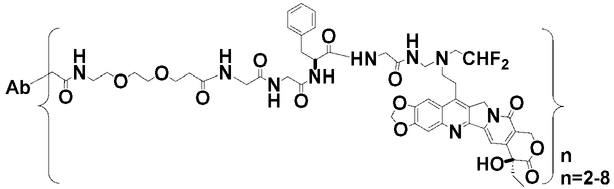 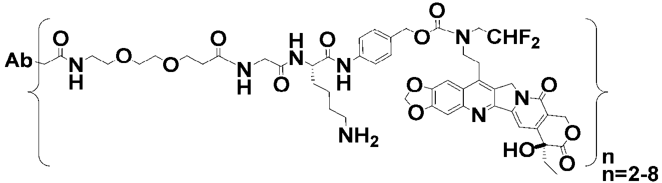 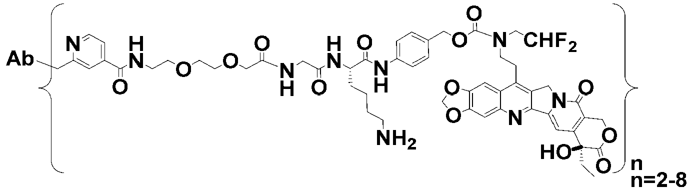 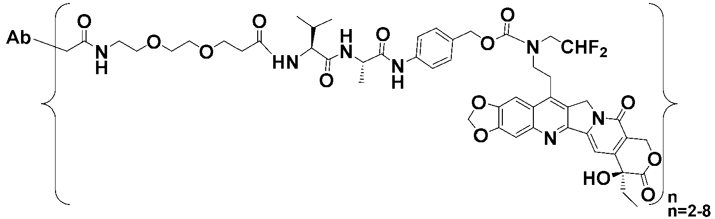 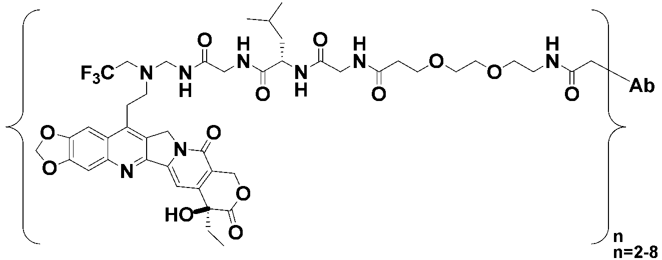 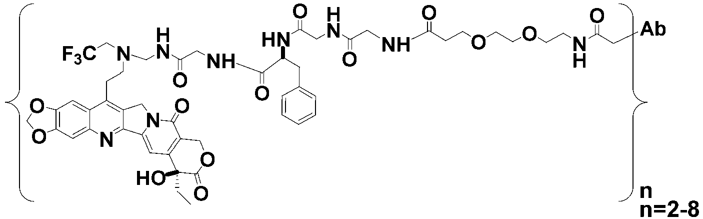 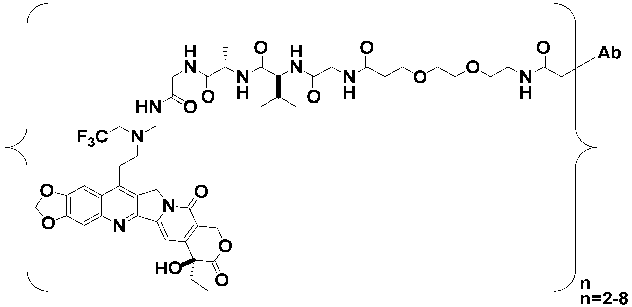 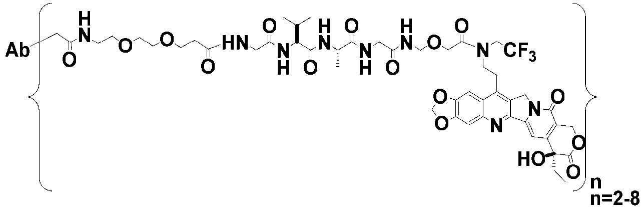 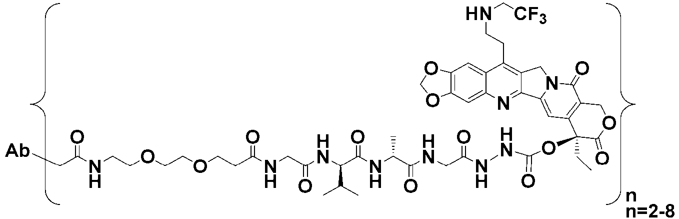 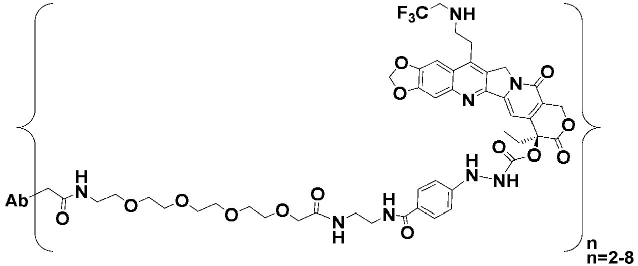 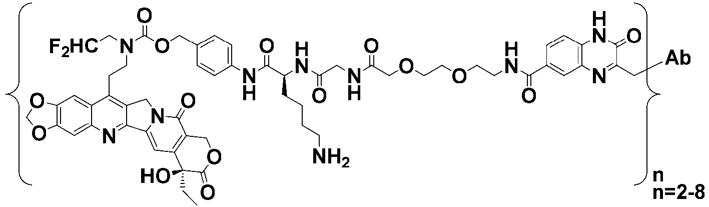 wherein, Ab is defined the same as the compound of Formula (I); preferably, Ab is HS627 antibody or IP140B antibody, the heavy chain amino acid sequence of HS627 is as set forth in SEQ ID NO: 1, and the light chain amino acid sequence thereof is as set forth in SEQ ID NO: 2; the heavy chain amino acid sequence of the IP140B antibody is as set forth in SEQ ID NO: 3, and the light chain amino acid sequence thereof is as set forth in SEQ ID NO: 4.

9. A pharmaceutical composition comprising the antibody-drug conjugate according to any one of claims 1-8 and a pharmaceutically acceptable carrier.

10. Use of the antibody-drug conjugate according to any one of claims 1-8 or the pharmaceutical composition according to claim 9 in the manufacture of an anti-tumor medicament;
preferably, the tumor is selected from a solid tumor, more preferably selected from esophageal cancer, lung cancer, non-small cell lung cancer, esophageal squamous cell carcinoma, ovarian cancer, lung cancer or breast cancer.

11. A method for treating a tumor disease, comprising the step of administering to a patient in need thereof the antibody-drug conjugate according to any one of claims 1-8 or the pharmaceutical composition according to claim 9.
